# EUROPEAN PATENT SPECIFICATION

(11) **EP 4 465 886 B1**
(45) Date of publication and mention of the grant of the patent: **19.08.2026**
(21) Application number: 23707623.7
(22) Date of filing: 27.01.2023
(51) Int. Cl.: A61B 5/12

(54) **SYSTEMS AND METHODS FOR ESTIMATING HEARING LOSS USING WIDEBAND ACOUSTIC IMMITTANCE**
SYSTEME UND VERFAHREN ZUR SCHÄTZUNG VON HÖRVERLUST UNTER VERWENDUNG VON BREITBANDIGER AKUSTISCHER IMMITTANZ
SYSTÈMES ET PROCÉDÉS D'ESTIMATION DE LA PERTE AUDITIVE À L'AIDE D'UNE IMMITTANCE ACOUSTIQUE À LARGE BANDE

(30) Priority: 28.01.2022 US 202263267271 P
(43) Date of publication of application: 27.11.2024
(73) Proprietor: Father Flanagan's Boys' Home doing Business as Boys Town National Research Hospital, Omaha, NE 68131 (US)
(72) Inventor: NEELY, Stephen Taylor, Omaha, Nebraska 68131 (US); MERCHANT, Gabrielle, Omaha, Iowa 68131 (US)
(74) Representative: Tomkinson, Alexandra
(86) International application number: PCT/US2023/061429
(87) International publication number: WO 2023/147458

(56) References cited:
- US-A1- 2019 038 189
- PRIEVE BETH A. ET AL: "Prediction of Conductive Hearing Loss Using Wideband Acoustic Immittance", EAR AND HEARING : OFFICIAL JOURNAL OF THE AMERICAN AUDITORY SOCIETY, vol. 34, no. Supplement 1, 1 July 2013 (2013-07-01), US, pages 54s - 59s, XP093046404, ISSN: 0196-0202, DOI: 10.1097/AUD.0b013e31829c9670
- EMAD M GRAIS ET AL: "Analysing Wideband Absorbance Immittance in Normal and Ears with Otitis Media with Effusion Using Machine Learning", ARXIV.ORG, CORNELL UNIVERSITY LIBRARY, 201 OLIN LIBRARY CORNELL UNIVERSITY ITHACA, NY 14853, 4 March 2021 (2021-03-04), XP081906046
- SACKMANN BENJAMIN ET AL: "Model-based hearing diagnostics based on wideband tympanometry measurements utilizing fuzzy arithmetic", HEARING RESEARCH, vol. 378, 28 February 2019 (2019-02-28), pages 126 - 138, XP085704765, ISSN: 0378-5955, DOI: 10.1016/J.HEARES.2019.02.011
- MERCHANT GABRIELLE R ET AL: "The influence of otitis media with effusion on middle-ear impedance estimated from wideband acoustic immittance measurements", THE JOURNAL OF THE ACOUSTICAL SOCIETY OF AMERICA, AMERICAN INSTITUTE OF PHYSICS, 2 HUNTINGTON QUADRANGLE, MELVILLE, NY 11747, vol. 150, no. 2, 6 August 2021 (2021-08-06), pages 969 - 978, XP012258736, ISSN: 0001-4966, [retrieved on 20210806], DOI: 10.1121/10.0005822

## Description

### CROSS REFERENCE TO RELATED APPLICATIONS

This application claims priority under 35 U.S.C. § 119 to provisional patent application U.S. Serial No. 63/267,271, filed January 28, 2022.

### STATEMENT REGARDING FEDERALLY SPONSORED RESEARCH OR DEVELOPMENT

This invention was made with government support under Grant No. P20GM109023 awarded by the National Institute of Health (NIH), as well as Grant Nos. R01DC008318 and L30DC017300 awarded by NIH. The government has certain rights in the invention.

### FIELD OF THE INVENTION

The invention relates generally to a system, apparatus, and/or corresponding method for the determination and/or estimation of hearing loss. More particularly, but not exclusively, the invention relates to systems and/or methods for the prediction of conductive hearing loss using wideband acoustic immittance data.

### BACKGROUND OF THE INVENTION

Otitis media (OM) is the most common reason children receive antibiotics, visit physicians, and have surgery (NIDCD). OM affects over 8 million children in the US each year and costs 5-billion dollars annually to treat. Common management of OM includes watchful waiting, antibiotics, and surgical placement of tympanostomy tubes. The optimum choice of treatment depends, at least in part, on specifics of the disease, as OM has numerous characteristics that can vary across individuals. These characteristics include presence or absence of acute or chronic infection, effusion features (e.g., viscosity and volume), and the impact of OM on hearing. However, there is currently a deficiency in tools to determine many of these characteristics objectively and accurately, and there are limitations to the tools commonly used. For example, clinical practice guidelines for OM predicate the need for surgery based on hearing levels (e.g., tympanostomy tube placement surgery), yet failure to obtain audiometric testing pre-operatively is common, likely due to difficulties in audiometric testing in the age range where OM is most prevalent (6-24 months). The lack of methods to differentiate and quantify various characteristics of OM leads to uncertainty and inefficiency regarding the most appropriate treatment.

Therefore, there exists a need in the art for an alternative method to estimate the degree of CHL in children with otitis media.

Limitations of the current methods for determining characteristics of OM also limit understanding of its long-term consequences. OM has been associated with deficits in speech, language, and auditory processing (including binaural processing), even after resolution of the disease. However, findings from studies are variable, resulting in a persistent debate regarding the long-term sequelae of OM. Equivocal outcomes are likely due to the heterogeneity of OM. OM represents a continuum of disease and thus has a variable impact on hearing. Systematic documentation of variables related to OM in previous studies -- such as degree of conductive hearing loss (CHL), effusion characteristics, time course, and presence of infection -- is limited. The magnitude and duration of auditory deprivation for children with permanent hearing loss have been associated with long-term negative outcomes in speech and language development. It is believed that a similar mechanism (i.e., the magnitude and duration of transient CHL) drives long-term sequelae in children with OM. However, while current objective diagnostic techniques can often indicate the presence or absence of OM, they do not inform about individual variations in OM that influence magnitude and duration of transient CHL. Understanding how variations in OM across the continuum are associated with CHL and long-term sequelae would help identify children at higher risk for persistent deficits and better inform treatment recommendations.

Treatment for OM varies widely and includes periodic monitoring of middle-ear status (watchful waiting), prescription of antibiotics, and surgical insertion of tympanostomy tubes. OM treatment is guided by clinical practice guidelines. However, adherence to OM clinical practice guidelines is variable and is as low as under 10% among primary care physicians and ENT specialists. This may be because, even with these guidelines, uncertainty still exists regarding the most effective treatment for a given episode of OM, as there is a lack simple, objective methods to determine diagnostic features of OM that are critical to prognosis and treatment success. Diagnosis is based largely on clinical presentation and symptoms combined with a subjective visual exam (i.e., otoscopy), sometimes in conjunction with tympanometry and audiometry. Such methods have not advanced in decades. OM is generally characterized by middle-ear effusion, either as acute otitis media (AOM) with acute inflammatory infection or lack thereof as in otitis media with effusion (OME). Children with AOM may exhibit signs of infection including fever and ear pain, as well as redness and swelling on the tympanic membrane (TM), but signs of infection are not always present or easy to determine. Even crying alone can cause redness of the TM. Thus, distinguishing AOM from OME can be challenging. Additionally, current diagnostic methods generally cannot determine individual variations in OM effusion characteristics. Effusion may be serous, mucoid, or purulent, and vary in both viscosity and volume. OM is also often accompanied by a fluctuating and transient CHL. Thresholds in children with OM can range from 0 to 40 dB HL. However, the OM-related factors determining the presence and degree of CHL are not well understood.

Variation in effusion volume, viscosity, and presence of infection likely impact the presence and degree of CHL. OM increases low-frequency stiffness resulting in a decrease in low-frequency TM mobility. Factors implicated in this observed stiffness increase include a reduction in the air in the middle-ear space, abnormal middle-ear static pressure, and reduced ossicular mobility. As the effusion adds mass to the TM, it decreases high-frequency TM mobility. Individual variations in OM characteristics can influence how OM alters the mass and stiffness properties of the system. For example, variation in effusion volume could differentially alter the amount of air in the middle-ear space and the mass on the TM. Variation in effusion viscosity and purulence could impact both stiffness-related ossicular mobility and the degree of the change in mass in different ways. These mechanical variations, in turn, likely influence presence and degree of CHL. Accurate diagnosis of OM and differential diagnosis of AOM versus OME are crucial in guiding management. OME does not have an infectious process that can be treated with antibiotics, so differentiating cases of OME from AOM is critical to avoid the use of unnecessary antibiotics. In contrast, watchful waiting (as opposed to tubes) in cases of chronic OME could increase the risk of long-term sequelae, such as deficits in auditory processing and speech and language development. Certain features of a given episode of OM may also influence the overall prognosis (i.e., whether the effusion will clear without intervention, as may be the case with higher viscosity or volume effusions). Our lack of objective diagnostic tools to determine effusion types limits scientific-based treatment decisions to manage different forms of OM.

Current assessment of OM includes otoscopy and tympanometry. These measures can often (but not always) determine the presence or absence of OM but provide limited further differentiation. Diagnosis and interpretation accuracy of otoscopy is subjective and highly variable, with a sensitivity ranging from 40-70%. Gaining a clear unobstructed view of the TM in children is not always possible. In addition, when there is no active infection, signs of OM are not always obvious. Tympanometry is often used to identify OM more objectively with a sensitivity and specificity of 86% and 72%, respectively. However, tympanometry does not provide detailed information on middle-ear mechanics as it mainly provides stiffness-dominated information at a single acoustic frequency. Importantly, abnormal tympanometry is only weakly associated with presence and degree of CHL. The limitations in the differentiation that current diagnostic tools provide result in a lack of specificity as to what treatment may be most effective and demonstrate the need for improved diagnostics for OM.

In addition to differentiating and characterizing OM related variables, the development of methods to objectively predict the impact of a given episode of OM on hearing is important. OM guidelines predicate the need for tube surgery based on hearing levels. Unfortunately, there are limitations in the feasibility of behavioral testing in the age range where OM is most common (6-24 months). A yield rate of 60-70% has been reported for the measurement of hearing sensitivity in 2-year-olds (an age with a high incidence of OM) using conditioned-play audiometry, and that is in the sound field, where ear-specific information isn't even obtained. Yield rates for ear-specific audiograms are even lower. While measuring over multiple test sessions may be feasible for documentation of permanent hearing loss, this approach is problematic in children with OM, due to fluctuating loss. Completing behavioral testing in children who are not feeling well is also challenging. This is likely why failure to obtain audiometric testing pre-operatively prior to tube placement is common, despite guideline recommendations. As such, there is a critical need for an alternative method to estimate the degree of CHL.

Wideband acoustic immittance (WAI) will improve the differential diagnosis of OM. WAI is a promising non-invasive measure of middle-ear mechanics. WAI refers to a group of transfer functions that may be derived from non-invasive and objective ear-canal acoustic measurements in response to wideband stimuli (as opposed to narrow-band or single-frequency stimuli). WAI refers to a group of acoustic measurements that provide detailed mechanical and acoustic information (i.e., absorbance, power reflectance, admittance, and impedance, among others). WAI measurements are made in the ear canal in response to wideband stimuli (e.g., a click or chirp) and compare a sound input to the absorbed or reflected portions of that sound. Changes in acoustic impedance due to pathological alterations in auditory mechanics influence WAI. In contrast to standard tympanometry, WAI allows for detection of middle-ear mechanical effects on both the mass and stiffness properties of the middle ear across a wide range of frequencies. WAI has been shown to be sensitive to the presence or absence of middle-ear effusion in infants and children with OM, in temporal bone simulations of middle-ear effusion, and in animal models of AOM.

WAI is measurable with a device that is FDA-approved for clinical use, but adoption of WAI has been limited, likely due in part to challenges in interpreting complex output data. Clinical viability would be significantly increased by not only understanding how pathology alters WAI, but also by developing algorithms to simplify and/or automate diagnostic interpretation.

Transfer functions that may be derived from ear-canal acoustic measurements include impedance, admittance, and reflectance. These transfer functions are collectively known as wideband acoustic immittance (WAI) and have been demonstrated to have clinical utility in the assessment of middle-ear status, particularly in differentiating origins of conductive hearing loss, including both middle- and inner-ear causes. WAI measures are clinically informative because acoustic admittance at the tympanic membrane is affected by pathological alterations in middle- and inner-ear mechanics.

Reflectance is defined as the transfer function between forward and reverse components of ear-canal pressure. Reflectance magnitude is expected by theory to be insensitive to the location within the ear canal of the probe tip when the ear canal is assumed to be a rigid tube that has negligible variation in cross-sectional area. However, actual ear canals have more complex geometry. Therefore, a more direct estimate of middle-ear admittance would useful information about the mechanisms underlying reflections.

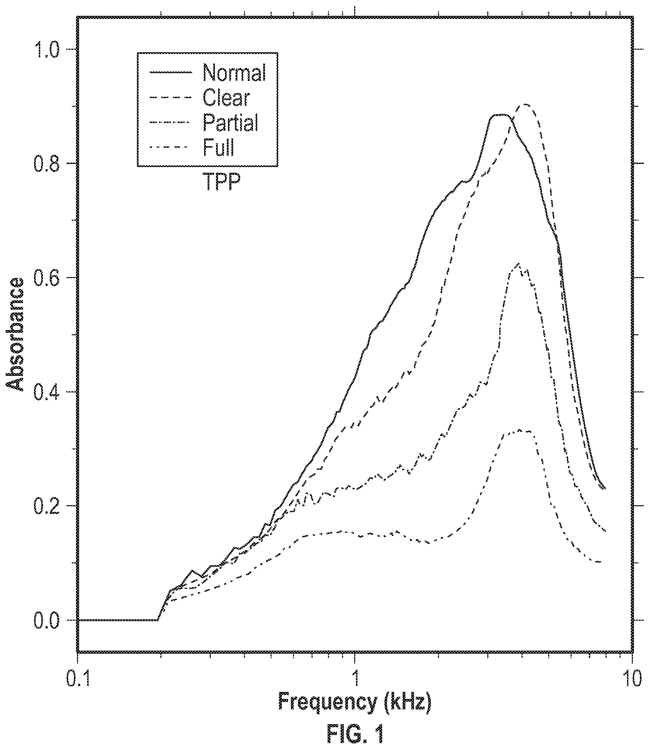

In at least one recent study, acoustic measurements in ears diagnosed as having otitis media with effusion (OME) were grouped as being full, partially full, or clear based on effusion volume observed at the time of tube surgery. The same acoustic measurements were made in age-matched healthy ears for comparison. The influence of effusion volume on absorbance, which is defined as one minus reflectance-magnitude squared, is shown in Fig. 1. Absorbance tends to decrease with increasing effusion volume.

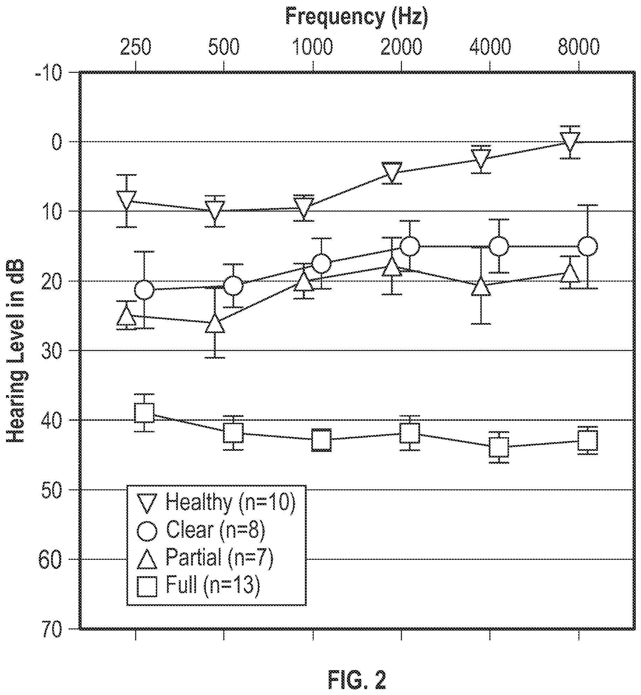

In a subset (n=34) of these OME ears, audiometric thresholds were also obtained. Average thresholds across each of the effusion groups are shown in Fig. 2. The difference in hearing level between audiologically-normal ears and OME ears is presumably due mostly to conductive hearing loss (CHL) caused by the presence of fluid in the middle ear. Note in Fig. 2 that, CHL tends to increase with effusion volume.

Estimates of CHL may depend on whether the cochlea detects sound pressure or sound power at the threshold of hearing. The fact that the primary sensory cells of hearing (i.e., hair cells) are essentially displacement detectors suggest that hearing thresholds should be sensitive to cochlear pressure at the threshold of hearing. Further support for this view comes from the fact that forward pressure level was observed to have better correlations with hearing threshold compared to transmitted power. On the other hand, general principles of communication theory suggest that the optimal receiver would detect power at threshold.

While there are devices that are used to make WAI measurements, these are limited in the data that they record, as well as how that data is utilized. For example, the data is not readily usable for application of determining audiological issues, such as CHL. PRIEVE BETH A. ET AL: "Prediction of Conductive Hearing Loss Using Wideband Acoustic Immittance", EAR AND HEARING : OFFICIAL JOURNAL OF THE AMERICAN AUDITORY SOCIETY, vol. 34, no. Supplement 1, 1 July 2013 (2013-07-01), pages 54s-59s, US, ISSN: 0196-0202, DOI: 10.1097/ AUD.0b013e31829c9670 relates to WAI measurements.

Thus, there exists a need in the art for systems, apparatus, and/or corresponding methods that utilizes individual ear-canal acoustic measurements, such as WAI, to predict CHL.

### SUMMARY OF THE INVENTION

The following objects, features, advantages, aspects, and/or embodiments, are not exhaustive and do not limit the overall disclosure. No single embodiment need provide each and every object, feature, or advantage. Any of the objects, features, advantages, aspects, and/or embodiments disclosed herein can be integrated with one another, either in full or in part.

It is a primary object, feature, and/or advantage of the invention to improve on or overcome the deficiencies in the art.

It is a further object, feature, and/or advantage of the invention to predict or estimate the amount of conductive hearing loss (CHL) in people, including, but not limited to, children with otitis media.

It is still yet a further object, feature, and/or advantage to collect data and to utilize the data for the prediction of CHL utilizing non-invasive acoustic measurement to collect data from a person.

It is another object, feature, and/or advantage to objectively estimate the amount of hearing loss in a child using a non-invasive measure that does not require the child to participate in the testing at all.

It is still another object, feature, and/or advantage to extract of information-rich features from wideband acoustic immittance measurements (WAI) based on modeling the underlying middle-ear mechanics.

It is yet another object, feature, and/or advantage to identify and predict two distinct types of inner ears.

It is still another object, feature, and/or advantage to estimate pathologies, conditions, and/or issues in an ear based upon a machine learned system that is trained using information gathered from modeled ear mechanics.

It is yet another object, feature, and/or advantage to identify and/or predict distinct types of inner ears, which can be utilized to determine an ear condition, such as CHL, OME, and other conditions.

The system, apparatus, and/or processes disclosed herein can be used in a wide variety of applications. For example, they can be implemented into a non-invasive tool that measures WAI data to more accurately predict CHL, as well as other ear conditions, pathologies, and other issues related to the ear, meaning they can be used in both clinical and research settings. For example, the algorithm could detect the presence of effusion volume, which could be used to diagnose OME and other conditions.

The systems and/or processes disclosed herein can be incorporated into systems and/or apparatus, such as non-invasive diagnostic tools, which accomplish some or all of the previously stated objectives.

According to at least some aspects of some embodiments disclosed herein, a method for estimating or predicting the amount of CHL caused by an episode of OME on an individual basis from non-invasive and objective ear-canal acoustic WAI measurements within a clinically meaningful margin of error is developed. A feature of this method is an estimate of acoustic admittance at the TM derived by fitting an electrical-analog model of ear-canal acoustics and middle-ear mechanics to measured absorbance. Absorbance and reflectance magnitude are expected by theory to be insensitive to the location within the ear canal of the probe tip when the ear canal is assumed to be a rigid tube that has negligible variation in cross-sectional area. However, actual ear canals have more complex geometry. Therefore, a more direct estimate of middle-ear admittance provides better information about the middle-ear mechanisms that influence acoustic reflections at the tympanic membrane and is achieved through our modeling approach. The success of our approach could directly influence clinical practice by potentially leading to an alternative protocol utilizing WAI for estimating the magnitude of CHL caused by OME in children, particularly when behavioral thresholds are either unable to be obtained and/or are found to be unreliable (due to a number of potential reasons, such as participant compliance and/or interest, lack of a play partner, equipment limitations, and/or limited time, among other possibilities).

According to at least some aspects disclosed, a method of estimating hearing loss comprises obtaining an acoustic measurement from an ear canal; modeling the acoustic measurement with an electric-analog model to obtain a model output; transforming the model output to a measured absorbance to determine an averaged absorbance over a frequency range converted to decibels; and training a machine learning network, wherein the training comprises: acquiring measured hearing loss data; fitting the parameters of the model such that the transformed model output correlates to the measured hearing data; and identifying one or more classifiers of the transformed model output that provides an estimate of the hearing loss.

According to at least some aspects of some embodiments, the acoustic measurement comprises an impedance-based measurement.

According to at least some aspects of some embodiments, the impedance based measurement comprises a wideband acoustic immittance.

According to at least some aspects of some embodiments, the step of modeling the acoustic measurement further comprises a transmission line representing the ear canal terminated by the network.

According to at least some aspects of some embodiments, the network of the model comprises three parallel branches, with each branch comprising a stiffness, damping, and mass component, wherein the network represents mechanics of a tympanic membrane coupled to ossicles of an ear.

According to at least some aspects of some embodiments, the averaged absorbance over a frequency range is compared to a pure tone average to obtain the estimated hearing loss.

According to additional aspects of the disclosure, a method of estimating hearing loss comprises obtaining an acoustic measurement from an ear canal; modeling the acoustic measurement with an electric-analog model to obtain model outputs including absorbance levels and ossicular loss levels in decibels; using a machine-learned network to identify ear type, wherein the machine-learned network comprises: comparing ossicular loss level outputs to a pure tone average to identify classifiers; and separating the classifiers into at least first and second ear types; based upon the identified ear type, transforming the model outputs to estimate the hearing loss.

According to at least some aspects of some embodiments, the classifiers comprise: a first identifier wherein the ossicular loss level outputs that are lower than the pure tone average; or a second identifier wherein the ossicular loss level outputs that are larger than the pure tone average by a factor of at least two.

According to at least some aspects of some embodiments, the step of transforming the model outputs comprises combining the absorbance levels and ossicular loss levels to obtain an estimated hearing loss for the first identifiers.

According to at least some aspects of some embodiments, the step of transforming the model outputs comprises dividing the ossicular loss levels by two to obtain an estimated hearing loss for the second identifiers.

According to at least some aspects of some embodiments, the estimated hearing loss is determined by comparing the results to a known pure tone average.

According to at least some aspects of some embodiments, the acoustic measurement comprises an impedance-based measurement.

According to at least some aspects of some embodiments, the impedance based measurement comprises a wideband acoustic immittance.

According to at least some aspects of some embodiments, the step of modeling the acoustic measurement further comprises a transmission line representing the ear canal terminated by the network.

According to at least some aspects of some embodiments, the network of the model comprises three parallel branches, with each branch comprising a stiffness, damping, and mass component, wherein the network represents mechanics of a tympanic membrane coupled to ossicles of an ear.

According to additional aspects of the disclosure, a system for estimating hearing loss comprises a device for obtaining an acoustic measurement from an ear canal; the device including a computer readable medium configured to: obtain the acoustic measurement from the ear canal; model the acoustic measurement with an electric-analog model to obtain model outputs and training a machine-learned network, wherein the training comprises: identifying values of the model output that correlate with measured or assessed data; based upon machine-learned training, identify a classifier indicating an ear type, said machine-learned training including the steps of comparing ossicular loss level outputs to a pure tone average; and based upon the identified ear type, transform the model outputs to estimate the hearing loss.

According to at least some aspects of some embodiments, the classifiers comprise: a first identifier wherein the ossicular loss level outputs that are lower than the pure tone average; or a second identifier wherein the ossicular loss level outputs that are larger than the pure tone average by a factor of at least two.

According to at least some aspects of some embodiments, the electric-analog model comprises a transmission line representing the ear canal terminated by the network.

According to at least some aspects of some embodiments, the network of the model comprises three parallel branches, with each branch comprising a stiffness, damping, and mass component, wherein the network represents mechanics of a tympanic membrane coupled to ossicles of an ear.

According to at least some aspects of some embodiments, the transmission line of the model represents an ear canal comprising a plurality of concatenated truncated-cone sections.

These and/or other objects, features, advantages, aspects, and/or embodiments will become apparent to those skilled in the art after reviewing the following brief and detailed descriptions of the drawings. Furthermore, the present disclosure encompasses aspects and/or embodiments not expressly disclosed but which can be understood from a reading of the present disclosure, including at least: (a) combinations of disclosed aspects and/or embodiments and/or (b) reasonable modifications not shown or described.

### BRIEF DESCRIPTION OF THE DRAWINGS

Several embodiments in which the invention can be practiced are illustrated and described in detail, wherein like reference characters represent like components throughout the several views. The drawings are presented for exemplary purposes and may not be to scale unless otherwise indicated.
Figure 1 is a graph showing mean absorbance at tympanometric peak pressure (TPP) across the four effusion volume groups: healthy normal control, OME full, OME partial, and OME clear.
Figure 2 is a graph showing average audiometric thresholds grouped by volume of effusion in children with otitis media.
Figure 3 is a schematic of an analog circuit model of ear canal acoustics and middle ear mechanics.
Figures 4A-4D are graphs showing measured and modeled mean absorbance based on effusion volume groups.
Figures 4E-4H are graphs illustrating differences between measured and modeled data for each effusion volume group.
Figure 5A is a graph showing the effect of effusion volume on middle-ear admittance for each of the four effusion volume groups.
Figure 5B is a graph showing the influence on the phase by the groups of effusion volume.
Figures 6A-6F show effect of effusion volume on the admittance of each branch of the middle-ear model.
Figure 7 is a graph showing absorbance levels of the effusion volume groups as a function of pure tone average.
Figure 8 is a graph showing ossicular loss levels of the effusion volume groups as a function of pure tone average.
Figure 9 is a graph showing the predicted hearing loss as a value of decibels as a function of pure tone average based upon aspects of the present disclosure.
Figure 10 shows graphs including umbo admittance modeled as the sum of two branch admittances, Y1+Y3.
Figure 11 shows graphs including umbo velocity.
Figure 12 is a schematic diagram showing steps of the development of a middle-ear conductive hearing loss algorithm.
Figure 13 is a schematic diagram showing steps of the application of a system or method for middle-ear conductive hearing loss estimation.
Figure 14 is a schematic diagram showing steps of the development of a middle-ear fluid volume determining algorithm.
Figure 15 is a schematic diagram showing the application of a system or method for diagnosing/estimating the amount of middle-ear fluid volume.

An artisan of ordinary skill need not view, within isolated figure(s), the near infinite number of distinct permutations of features described in the following detailed description to facilitate an understanding of the invention.

### DETAILED DESCRIPTION OF THE INVENTION

The present disclosure is not to be limited to that described herein. Mechanical, electrical, chemical, procedural, and/or other changes can be made without departing from the scope of the invention. No features shown or described are essential to permit basic operation of the invention unless otherwise indicated.

Unless defined otherwise, all technical and scientific terms used above have the same meaning as commonly understood by one of ordinary skill in the art to which embodiments of the invention pertain.

The terms "a," "an," and "the" include both singular and plural referents.

The term "or" is synonymous with "and/or" and means any one member or combination of members of a particular list.

The terms "invention" or "present invention" are not intended to refer to any single embodiment of the particular invention but encompass all possible embodiments as described in the specification and the claims.

The term "about" as used herein refer to slight variations in numerical quantities with respect to any quantifiable variable. Inadvertent error can occur, for example, through use of typical measuring techniques or equipment or from differences in the manufacture, source, or purity of components.

The term "substantially" refers to a great or significant extent. "Substantially" can thus refer to a plurality, majority, and/or a supermajority of said quantifiable variable, given proper context.

The term "generally" encompasses both "about" and "substantially."

The term "configured" describes structure capable of performing a task or adopting a particular configuration. The term "configured" can be used interchangeably with other similar phrases, such as constructed, arranged, adapted, manufactured, and the like.

Terms characterizing sequential order, a position, and/or an orientation are not limiting and are only referenced according to the views presented.

The "scope" of the invention is defined by the appended claims.

The term "pure tone average" (PTA) refers to the average of hearing threshold levels at a set of specified frequencies: 500, 1000, 2000 and 4000 Hz. This value gives a snapshot of an individual's hearing level in each ear. As speech sounds are more densely represented in the mid frequencies, the outlying frequencies are not included in the PTA calculation to allow for more consistent comparisons. If your PTA is <25 dB, your overall hearing would be considered to be within normal limits. With a PTA of 95 dB, your hearing would be considered in the profound range.

### Wideband acoustic immittance.

As described herein, multiple transfer functions or measurements or can be calculated from each WAI response. Reflectance Γ(*f*) is defined as the complex ratio of reverse pressure to forward pressure. In a uniform tube, reflectance is related to the input impedance at the point of measurement by the equation: $Γ \left(f\right) = \frac{{Z}_{ec} \left(f\right) - {Z}_{0}}{{Z}_{ec} \left(f\right) + {Z}_{0}} ,$
where *f* is frequency, *Z*_{ec}(*f*) is the frequency-dependent impedance looking into the ear canal, and *Z*₀ is the characteristic acoustic impedance of the ear canal at the point of measurement. The characteristic impedance is calculated as: ${Z}_{0} = ρ c / {A}_{ec}$

Absorbance is the recommended WAI measure form for clinical assessment. Absorbance 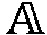 is defined as: $A \left(f\right) = 1 - {\left|Γ\left(f\right)\right|}^{2}$

Absorbance measurements averaged across each of the four OME groups (full, partial, clear, and normal) are shown in Fig. 1.

To stabilize the fitting of model parameters, noisy data below 0.2 kHz was replaced by extrapolation of measured ear-canal impedance. This smoothing procedure consisted of extending the real part of the ear-canal impedance at 0.2 kHz with the same value to all frequencies below 0.2 kHz and extending the imaginary part with its magnitude inversely proportional to frequency, which would be consistent with it being due to a compliance.

### Ear-canal and middle-ear model

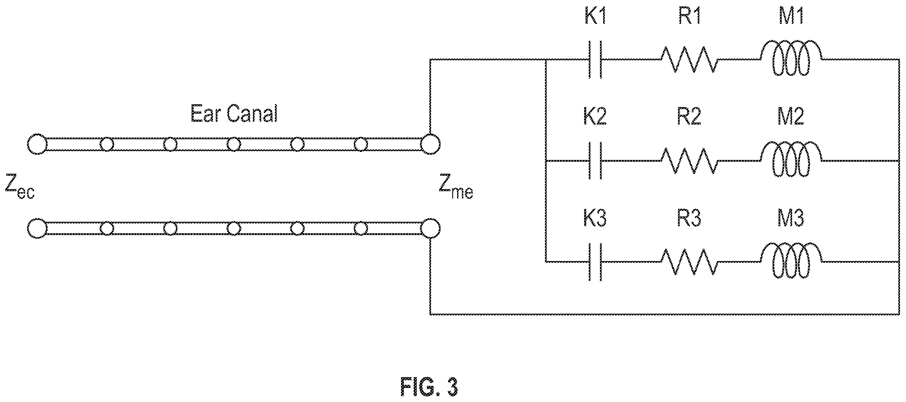

The analog circuit model that is used to represent ear-canal acoustic responses is shown in Fig. 3. This model comprises a nonuniform transmission line that is terminated by a network of stiffness (K), damping (R), and mass (M) components. The transmission line represents the ear canal, while the terminating circuit represents the middle ear. The nonuniform transmission line is modeled as six concatenated truncated-cone sections. The transmission line is implemented numerically as a lossless two-port transmission matrix.

As shown in Fig. 3, the ear-canal is modeled as a non-uniform transmission line comprising six concatenated segments. The middle-ear input impedance *Z*ₘₑ is modeled as three parallel branches that each contain stiffness K, damping R, and mass M components.

The middle-ear network contains three branches that together represent mechanics of the tympanic membrane coupled to the ossicles (i.e., malleus, incus, and stapes). Middle-ear input impedance *Z*ₘₑ is calculated as the parallel impedance of these three branches. ${Z}_{me} = \frac{1}{1 / {Z}_{1} + 1 / {Z}_{2} + 1 / {Z}_{3}} .$

In this equation, *Z*₁ = *jωM*₁ *+ R*₁ *+ K*₁/*jω*, *Z*₂ = *jωM*₂ *+ R*₂ + *K*₂/*jω*, and *Z*₃ *= jωM*₃ + *R*₃ *+ K*₃/*jω*, where *ω* = 2π*f*, and *f* represents frequency. The specific association of middle-ear components in the model with middle-ear anatomy is not required for estimation of CHL.

The model ear-canal input impedance *Z*_{ec} is related to its middle-ear impedance *Z*ₘₑ by elements of the ear-canal transmission matrix:

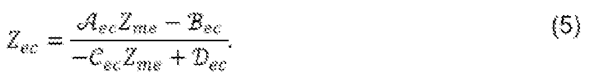

Model parameters were selected by a "simplex" search algorithm that minimizes a cost function that is calculated as a weighted sum of deviations between the model and measurements of ear-canal impedance and absorbance. Search constraints, which comprised mostly lower and upper bounds on the model parameters, were implemented by adding a constraint penalty to the cost function.

### Middle-ear admittance

An alternative to Eq. (4), is to calculate *Z*ₘₑ from ear-canal impedance *Z*_{ec} by, in effect, inverting Eq. (5):

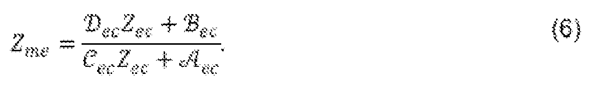

The matrix elements in Eq. (6) are the same as those in Eq. (5) because the transmission line is a reciprocal network, so the transmission matrix has a determinant that is equal to one, 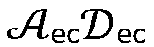 - 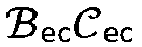 = 1. When *Z*_{ec} is measured instead of calculated from the model, then *Z*ₘₑ becomes an estimate of middle-ear impedance. Admittance is the reciprocal of impedance, so *Y*ₘₑ = 1/Zₘₑ. This notation may be extended as *Y*_{me,m} and *Y*_{me,e} to distinguish between the modeled and estimated versions of middle-ear admittance, respectively.

### Conductive hearing loss estimate

Two different approaches to modeling CHL will be considered. The first approach models CHL as being due to incomplete sound absorption (i.e., reflection) at the tympanic membrane and is calculated as the average absorbance 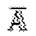 over the frequency range from *f*₁ = 2 kHz to *f*₂ = 4 kHz converted to decibels: $\overline{A} = \frac{1}{{f}_{2} - {f}_{1}} {\int }_{{f}_{1}}^{{f}_{2}} A \left(f\right) df ,$ ${CHL}_{1} = - 10 ⋅ {log}_{10} \overline{A} .$

In Eq. (8), the minus sign is needed to make *CHL*₁ positive because 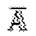 is always less than one.

The second approach models CHL as being due to a pressure drop across the ossicular chain, which is presumed to be in series with a cochlear load, and is calculated as the middle-ear model damping component *R*₁ divided by the cochlear load *R*₀ = 2 × 10⁷(mks acoustic ohm) converted to decibels: ${CHL}_{2} = 20 ⋅ {log}_{10} \left({R}_{1}/{R}_{0}\right) .$

This can be thought of as *ossicular* loss. The formulation of these two CHL estimates is meant to be suggestive of the underlying physics of the middle-ear. A third formula for estimating CHL will be derived below taking advantages of features that were observed when Eqs. (8-9) were applied to the OME data.

### Diagnostic prediction performance.

The four OME conditions (full, partial, clear, normal) may be predicted from acoustic measurements by utilizing binomial logistic regression (mnrfit, MATLAB) and hierarchical classification. A binomial logistic regression (often referred to simply as logistic regression) predicts the probability that an observation falls into one of two categories of a dichotomous dependent variable based on one or more independent variables that can be either continuous or categorical. A hierarchical classifier is a classifier that maps input data into defined subsumptive output categories. The classification occurs first on a low-level with highly specific pieces of input data. The classifications of the individual pieces of data are then combined systematically and classified on a higher level iteratively until one output is produced. This final output is the overall classification of the data. Depending on application-specific details, this output can be one of a set of pre-defined outputs, one of a set of on-line learned outputs, or even a new novel classification that hasn't been seen before. Generally, such systems rely on relatively simple individual units of the hierarchy that have only one universal function to do the classification. In a sense, these machines rely on the power of the hierarchical structure itself instead of the computational abilities of the individual components.

Prior to the regression, a principal component analysis reduced the decision variable, which was either absorbance or the magnitude of a middle-ear admittance, to fewer components. The hierarchical classification procedure first predicted whether effusion was present or absent. Then wet ears were classified as being either full or partial and dry ears as being either clear or normal. Test performance was quantified for each of the binomial categories by calculating the area under a receiver operating characteristic curve (AUC).

A fourth regression analysis was performed to predict whether *CHL*₂ over-estimates PTA by a factor of two, as will be understood as disclosed herein.

### Ear-canal and middle-ear model.

Model results were obtained by fitting model parameter values to measured ear-canal impedance Z*_{ec}* and absorbance. Separate model results were obtained at two different static pressures: 0 daPa (0Pa) and tympanic peak pressure (TPP).

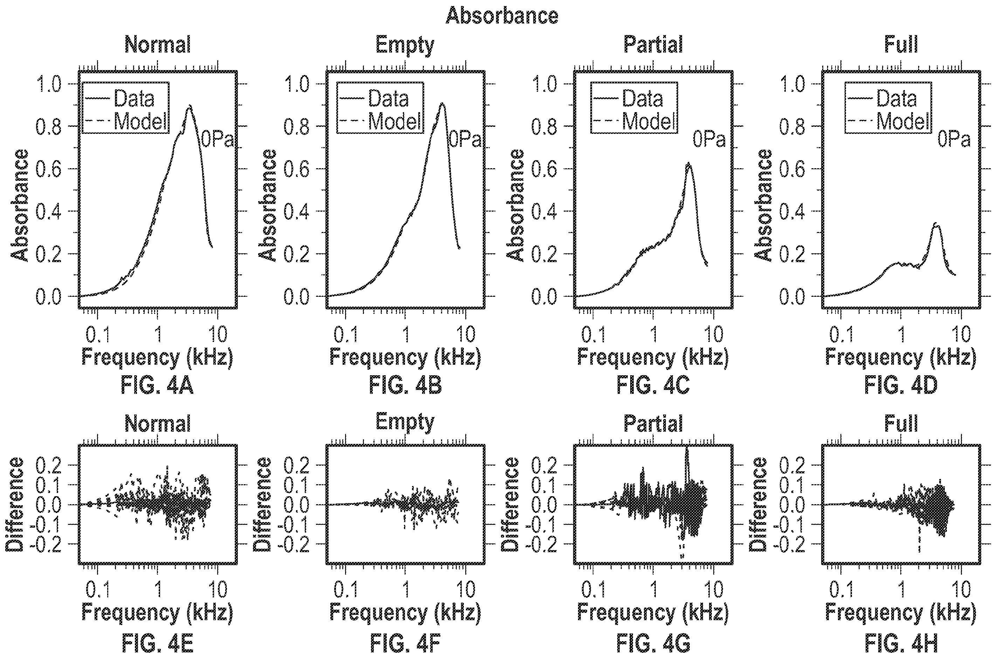

In the upper panels of Fig. 4, the model absorbances averaged across each group (red dashed) are superimposed over the average measured absorbance at TPP (blue solid). Individual differences between measured and modeled absorbance are shown in the lower panels for each individual ear included in that group, while the mean of those differences shown in black. On average, the agreement between the model and the measurements at TPP is excellent. Individual differences are mainly due to artifacts and "fine structure" in the measured absorbance.

The WAI measurement at TPP (i.e., absorbance) was selected for Fig. 4 to facilitate comparison with a similar figure known. Such comparison demonstrates that the current version of the middle-ear model fits the OME data at least as well as a previous version of the model that did not include M₃. The graphs of Fig. 4A-4H show results measured at 0Pa because CHL predictions were better. The top panels, Figs. 4A-4D, show measured (blue solid) and modeled (red dashed) mean absorbance. The bottom panels, Figs. 4E-4H show individual differences between measured and modeled data for each ear included in the mean above. The gray lines represent each individual difference, while the black line represents the mean difference. The columns represent the four OME conditions: normal, clear, partial, and full. For future reference, the nine middle-ear model parameters averaged across each OME group at 0Pa are listed in Table I.

Table I shows middle-ear model parameters at 0Pa averaged across each OME condition. For convenience, units of damping (R) are cgs acoustic ohm (dyn·s/cm⁵). Conversion to mks acoustic ohm (Pa·s/m³) requires multiplying tabled values by 10⁵. Units of mass (M) and stiffness (K) are multiplied and divided by 1 kHz, respectively.

**Table I: Middle-ear model parameters at 0Pa averaged across each OME condition.**

| OME condition | M₁ | R₁ | K₁ | M₂ | R₂ | K₂ | M₃ | R₃ | K₃ |
|---|---|---|---|---|---|---|---|---|---|
| full | 114 | 10792 | 21066 | 69 | 4274 | 171064 | 464 | 4324 | 14655 |
| partial | 90 | 5041 | 16720 | 68 | 2510 | 66403 | 389 | 2738 | 11209 |
| clear | 61 | 1319 | 11153 | 45 | 674 | 33447 | 361 | 3866 | 12194 |
| normal | 65 | 686 | 11377 | 34 | 873 | 25390 | 295 | 2832 | 9094 |

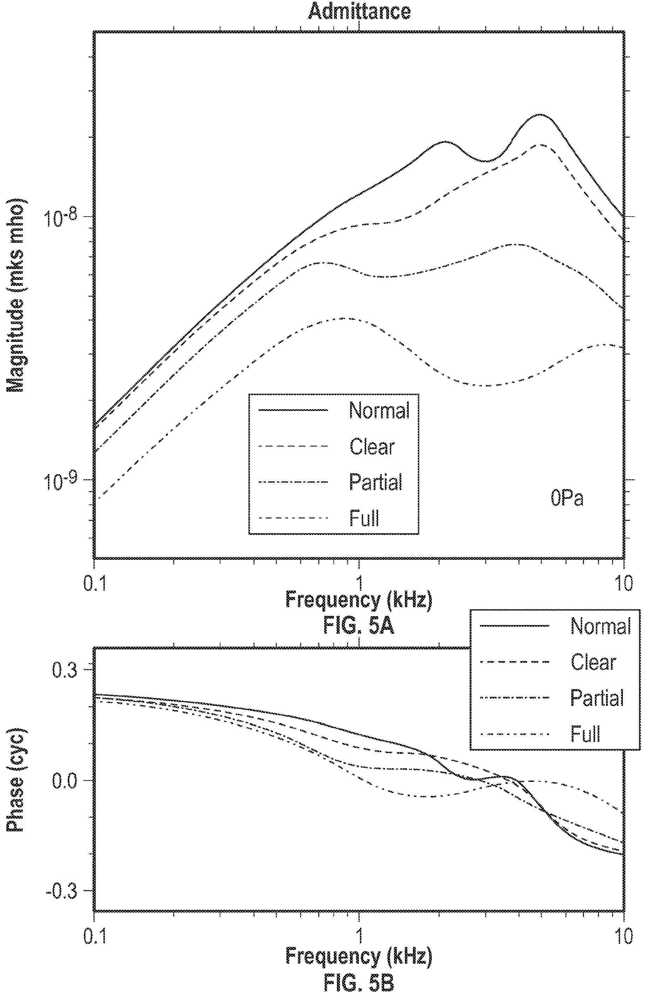

The effect of effusion volume on middle-ear admittance at 0Pa is shown in Fig. 5 by superimposing the average admittance for each of the four OME conditions. At frequencies below 0.8 kHz, where the admittance is dominated by stiffness, the partial and full conditions show an apparent increase in stiffness. Above 0.8 kHz, the main effect on admittance of increasing effusion volume is a magnitude decrease (upper panel). Concurrent flattening of the admittance phase (lower panel) above 1 kHz suggests that the magnitude decrease is likely due to increased damping. As shown, Fig. 5 shows the influence of effusion volume on middle-ear admittance at 0Pa. These admittances were calculated from the parameter values listed in Table I.

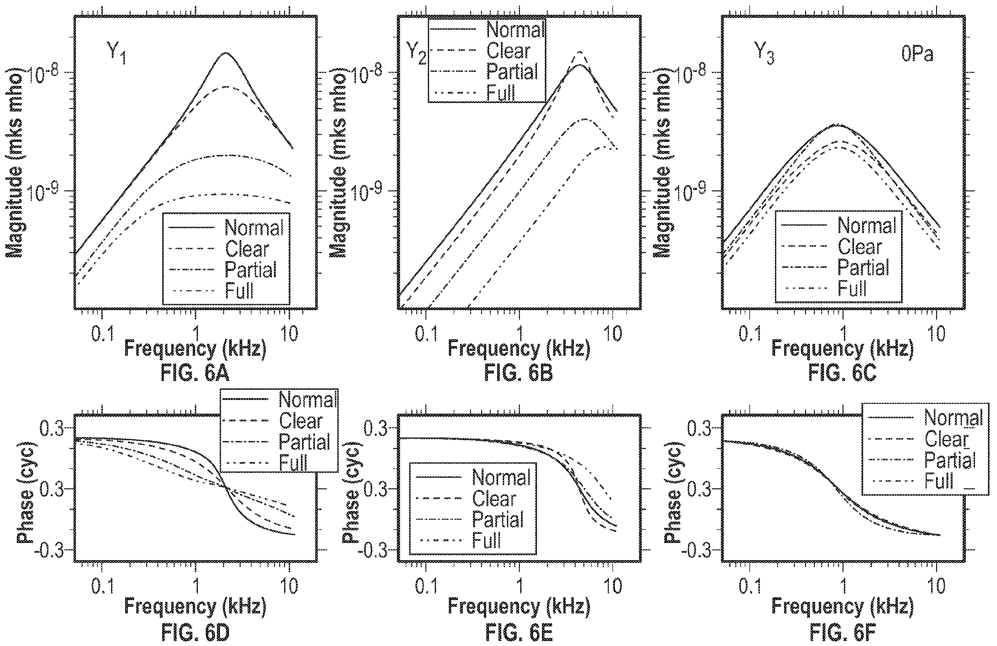

The effect of effusion volume on the admittance of each branch of the middle-ear model is shown in Fig. 6, which shows the effect of effusion volume on model-branch admittance at 0 daPa. These admittances were calculated from the parameter values listed in Table I. Because middle-ear admittance is calculated as the sum of the three branch admittances (*Y*ₘₑ = *Y*₁ + *Y*₂ + *Y*₃), Fig. 6 demonstrates a decomposition of tympanic motion into three distinct vibration modes. The main effect of effusion volume on Y₁ is an increase in damping. The main effect of Y₂ is an increase in both stiffness and damping. The effect on Y₃ is relatively small compared to the effects on Y₁ and Y₂.

### Conductive hearing loss estimate.

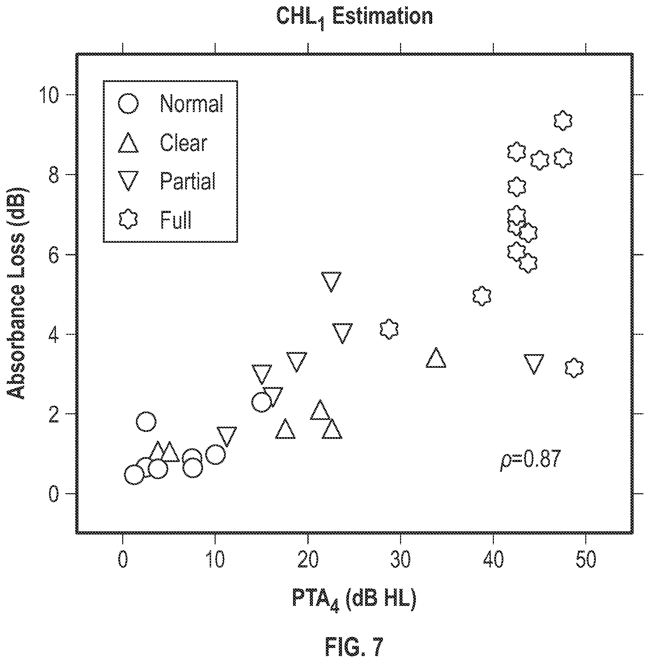

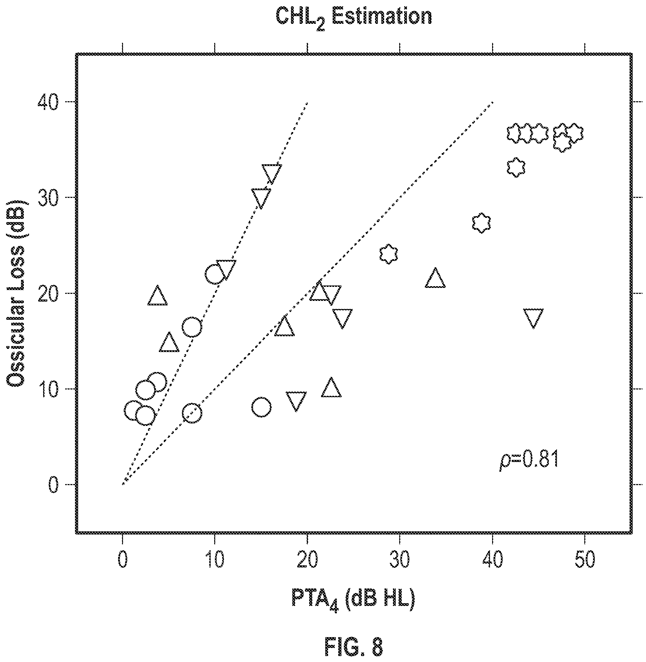

Individual absorbance level *CHL*₁ (left panel) and ossicular loss (right panel) *CHL*₂ are plotted in Figs. 7 and 8 as a function of pure tone average (PTA) for each of the 34 ears with audiograms. Both *CHL*₁ and *CHL*₂ are well-correlated with PTA (87% and 81% respectively). However, note that the range of absorbance loss is less than 10 dB, which is much less than the nearly 50 dB range of PTA. Although *CHL*₂ spans a larger (40 dB) range, it also falls short of explaining the entire PTA range. As included in Figs. 7 and 8, the correlation between each CHL estimate and PTA is labeled as *ρ.*

Note that *CHL*₂ (Fig. 8) appears to form two clusters that are separated by the two reference lines, which represent CHL slopes relative to PTA of one and two. The CHL estimates in the lower cluster (23/34) are consistently lower than PTA by as much as 10 dB. The CHL estimates in the upper cluster (11/34) are larger by than PTA a factor of two. This observation suggests a scheme for improving the CHL estimate. For ears in the lower cluster, *CHL*₁ (absorbance loss) will be added to *CHL*₂ (ossicular loss). For ears in the upper cluster, *CHL*₂ will be divided by two. Cluster assignment depends on whether *CHL*₂ is closer to PTA or PTA times 2. ${CHL}_{3} = \left\{\begin{matrix}{CHL}_{1} + {CHL}_{2} , & if lower cluster \\ {CHL}_{2} / 2 , & if upper cluster\end{matrix}.\right.$

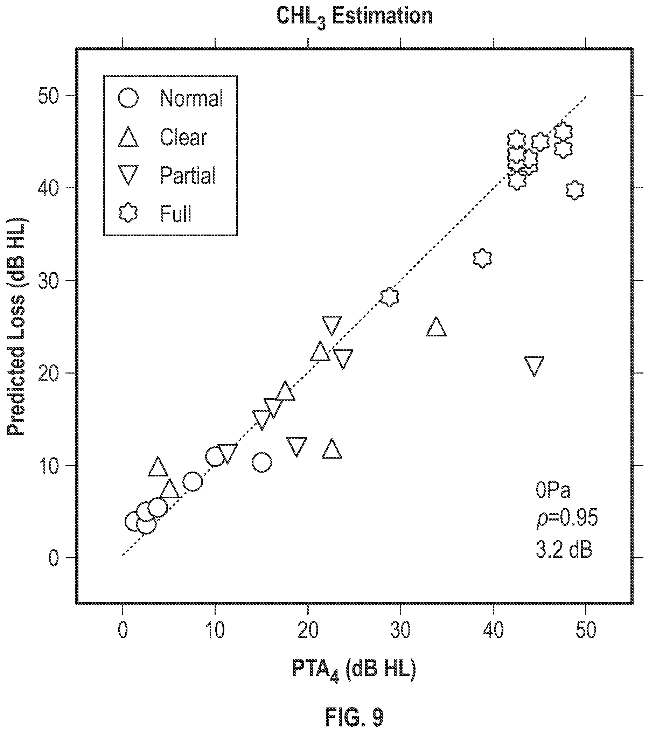

The results of applying Eq. (10) to the OME data are shown in Fig. 9, which shows a prediction of conductive hearing loss *CHL*₃ as a function of PTA. The is labeled as *ρ* and the estimation error is calculated as the mean absolute difference in dB. The correlation of *CHL*₃ with PTA is 95% and the prediction error, quantified as the mean absolute difference, is 3.2 dB. It is noted that 3.3dB is less than the +/- 5dB test retest reliability of pure tone behavioral audiometry. If we look at these results at TPP instead of 0Pa (not shown), the correlation of *CHL*₃ with PTA is reduced to 88% and the prediction error increases to 5.3 dB, which demonstrates the superiority of measurements at 0Pa for our CHL estimation methods.

Although Eq. (10) is empirically motivated, its application requires prior knowledge of whether an ear belongs to the lower or upper cluster. One way to address this requirement in practice would be to use a logistic regression to predict the appropriate cluster assignment from the WAI measurement, such as described in herein.

### Diagnostic prediction performance.

Diagnostic performance is quantified as the validation AUC (area under the curve) for each binomial hierarchical decision. In addition to the three hierarchical binomial decisions required for classification of OME group (effusion present or absent, effusion present ears as full/partial, effusion absent ears as clear/normal), a fourth binomial decision (ear type) is required to predict (at least without any a priori knowledge) whether the CHL2 cluster is on the lower or upper cluster.

For convenience, the present disclosure will refer to the lower and upper clusters by the designations LWR and UPR, respectively. To focus the training portion of the regression on these two clearly separated clusters, only 13 ears (8 LWR and 5 UPR ears), specifically those with a PTA in the 5 to 25 dB HL range, were included in the LWR/UPR regression analysis. Ears with a PTA below 5 dB were not included as there is no clear separation in that region of LRW and UPR ears. Ears with a PTA above 359 25 dB were not included as they are all LWR ears.

Diagnostic performance was optimized by selection of (1) a decision variable (A, |*Y*_{me,m}| |*Y*₁|, |*Y*₂|, |*Y*₃|, |*Y*₁ + *Y*₂|, |*Y*₁ + *Y*₃|, |*Y*₂ + *Y*₃|), and (2) a static pressure (0Pa or TPP) to achieve the largest possible AUC. For all regressions, the number of principal components was 3 and the regularization parameter (to avoid overfitting) was *λ* = 0.5. The four rows in Table II indicate the optimal combination of decision variable, static pressure, and number of principal components for each of the four binomial-decision types.

Table II below provides diagnostic prediction performance. The bold numbers indicate the maximum validation AUC for each of the four binomial-decision types.

**Table II: Diagnostic prediction performance.**

| Decision Variable | Static Pressure | Wet/ Dry | Full/ Partial | Clear/ Normal | LWR/ UPR |
|---|---|---|---|---|---|
| 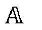 | TPP | **0.997** | 0.981 | 0.692 | 0.809 |
| \|*Y*₁ + *Y*₂\| | 0Pa | 0.988 | **0.999** | 0.720 | 0.669 |
| \|*Y*₁ + Y₃\| | TPP | 0.984 | 0.869 | **0.822** | 0.638 |
| \|*Y*₂ + *Y*₃\| | TPP | 0.917 | 0.875 | 0.650 | **0.992** |

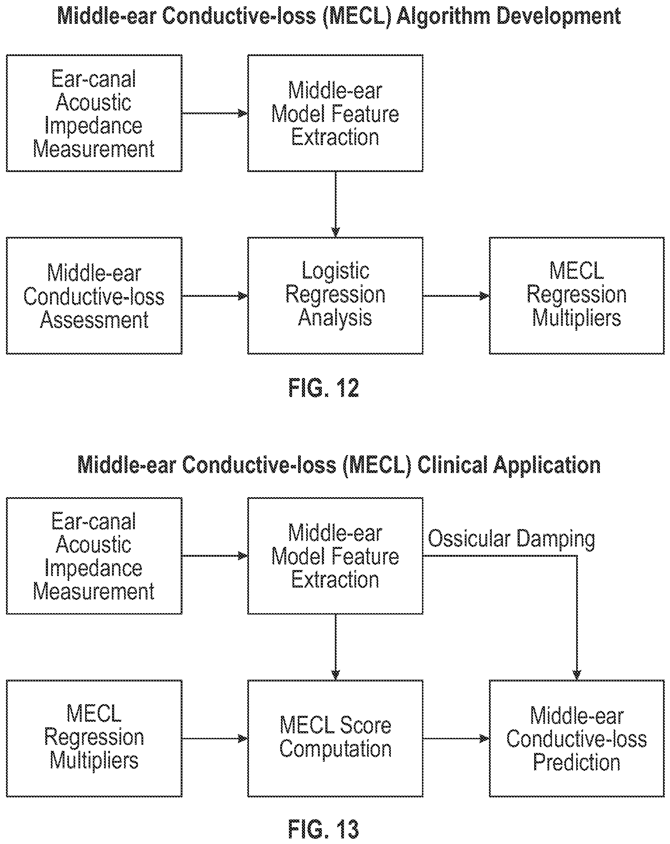

As disclosed herein, aspects and/or embodiments of the disclosure include the use of modeling, such as machine learning to perform steps and/or identify classifiers for the various steps of the processes provided. Examples of the processes for the machine learning and algorithms used for the aspects are shown generally in Figs. 12-15.

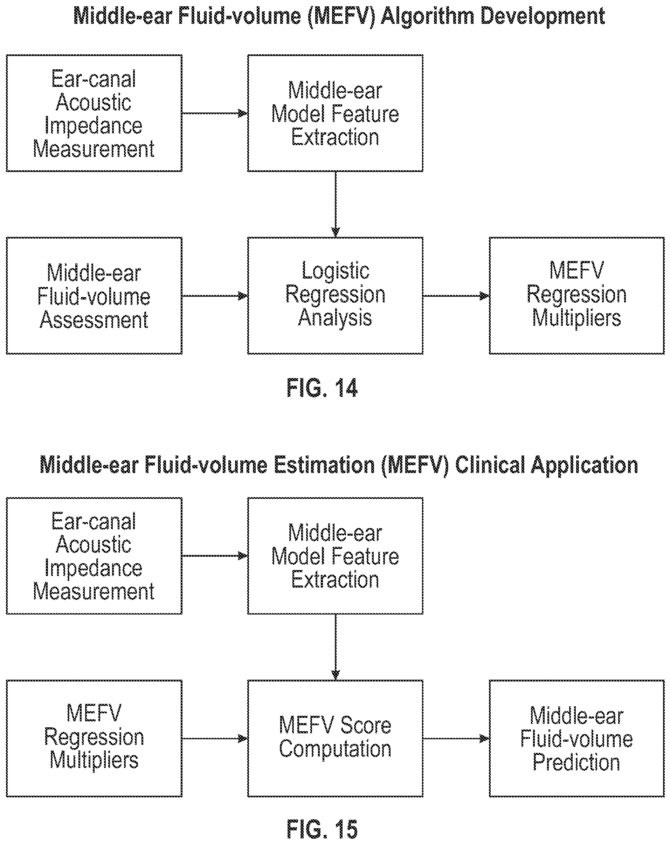

For example, in Fig. 14 a first box shown includes the acquisition of ear-canal acoustic impedance measurements, such as in the form of the WAI using a tool. As shown by the arrow, the next step involves a middle-ear feature extraction. This can be in the form of the electric-analog model, which is shown in Fig. 3. As noted herein, the model is used to transform the data from the acoustic measurements to usable data, such as data involving absorbance, power reflectance, admittance, and impedance, among others. The model is used to fit the data to mimic the ear canal and middle-ear mechanics, which will provide information akin to that of the actual measurements of an ear-canal and middle-ear.

A middle-ear fluid-volume (MEFV) assessment is done by measuring the fluid volume of a number of patients to acquire data on the patients, such as types of data that could be used to identify the amount of fluid volume. Model results were obtained by fitting model parameter values to measured ear-canal impedance and absorbance. As has been disclosed herein, the analog model and fitted parameter values that were used provides excellent overlap and agreement between the modeled values and the measured values.

Next steps include the estimation of effusion volume in an ear. This is done, for example, with machine learning of the assessed data and the acoustic data, wherein a regression model is utilized to train a network to determine classifiers. In the instant case, this may be in the form of admittance, which will identify classifiers that identify the listed ear types, including full effusion, partial effusion, ears clear of effusion, and normal ears. The logistic regression analysis will be used to identify middle-ear fluid-volume regression multipliers, which is an estimation of the fluid volume in an ear. To do so, the fitted parameters of the analog model are compared to assessed measurements to identify which parameters are key for determining/estimating MEFV. This results in regression multipliers, which are used as a basis for the estimation of MEFV.

In Fig. 15, a middle-ear fluid-volume estimation is provided that can be used in a clinical setting. Once the MEFV regression multipliers have been identified using the machine learning and logistic regression analysis shown in Fig. 14, said multipliers could be used in a diagnostic tool to quickly and accurately determine and estimate an amount of middle-ear fluid volume (MEFV). As shown in Fig. 15, the ear-canal acoustic impedance measurement is again used to acquire acoustic data. The middle-ear model feature extraction includes the electric-analog model to fit the acoustic data (i.e., the WAI data) to an actual ear-canal and middle-ear mechanics. The MEFV regression multipliers that have been trained are applied to the feature extraction information that has been modeled, and a score computation is developed. The machine learned classifiers are applied to the fit data from the WAI instrument, and a MEFV prediction is provided. Therefore, the modeling of the ear-canal and middle-ear mechanics can transform the acquired acoustic data, such as the WAI data, to be useful for clinical settings. The WAI tool can include the machine learned neural network that has been trained using the analog model of the ear and can provide quick and accurate predictions as to the middle-ear fluid-volume amounts.

This information will allow for a quicker and more objective source of information (i.e., the presence and amount of fluid in the middle ear) that can be used by clinicians to assess a patient's issues and to better diagnose the potential issues. In addition, as the WAI data acquisition is generally considered non-invasive (e.g., a probe tip is quickly inserted into an ear canal), the ability to assess and diagnose patients of all ages and conditions can be done in a quick, efficient, and accurate manner which improves over the common manner today, which is perform a number of invasive and/or highly subjective tests to verify an amount of fluid or other issues in the ear.

Additional algorithms are shown schematically in Figs. 12 and 13. For example, as shown in Fig. 12, a middle-ear conductive loss (MECL) algorithm is developed. As shown schematically, this includes first acquiring ear-canal acoustic impedance measurements, such as in the form of the WAI using a tool. Again, the data is transformed and fit to an ear-canal and middle-ear model, such as using the analogy model and steps related to Fig. 3 herein. This puts the steps at the middle-ear model feature extraction step.

The MECL assessment is done by measuring the hearing loss of a number of patients to acquire data on the patients, such as types of data that could be used to identify and quantify the hearing loss. Model results were obtained by fitting model parameter values to measured ear-canal impedance and absorbance. As has been disclosed herein, the analog model and fitted parameter values that were used provides excellent overlap and agreement between the modeled values and the measured values.

Again, a logistic regression analysis (e.g., a machine learning network) can be used to identify MECL regression multipliers that correlate the middle-ear model feature extraction data, which includes the fitted parameters of the analog model, with the assessed/measured data from patients to identify regression multipliers, also known as classifiers. In the present disclosure, the analysis was done two ways. The first approach (CHL₁) models CHL as being due to incomplete sound absorption (i.e., reflection) at the tympanic membrane and is calculated as the average absorbance from 2-4 kHz converted to decibels (dB). The second approach (CHL₂) models CHL as being due to a pressure drop across the ossicular chain, which is presumed to be in series with a cochlear load and is calculated as the middle-ear model damping component R1 divided by the cochlear load R₀=2×10⁷ (mks acoustic ohm) converted to dB.

However, as identified herein, a third approach included the identification of the classification of ear types, identified as the lower cluster (LWR) in Fig. 8, and those identified as the upper cluster (UPR) in Fig. 8. Therefore, the algorithm of Fig. 12 may be used to train a machine learning system to identify the classification of ear types, which are LWR and UPR.

Fig. 13 then shows a clinical application of the estimation of MECL at a clinic or in real life application. As shown in the figure, a WAI tool is used to acquire acoustic data, such as ear-canal acoustic impedance measurements. The analog model is used to fit the parameters of the ear-canal and middle-ear mechanics.

The regression multipliers (also referred to as classifiers) that were learned in Fig. 12, are applied to the data from the WAI tool. This includes the ossicular damping, which was identified as CHL₂ in the disclosure. The use of the ear types is applied to the data as provided for CHL₃, wherein the data is either added (CHL₁ + CHL₂) when the ear types are in the LWR cluster, or wherein the data is divided by two (CHL₂/2) when the ear types are in the UPR cluster. The resulting data of CHL₃ can then be plotted as a function of PTA to provide a quick and accurate estimation of the conductive hearing loss of the patient.

Subjects with OME and PTA measurements in both ears almost always had either two LWR ears or two UPR ears. The only exception had partial fluid in the UPR ear and full fluid in the LWR ear, which might suggest that the full-fluid condition interferes with UPR status. Indeed, none of the ears classified as being full had UPR status. The strong within-subject correlation of UPR status suggests that this feature may have a biological origin.

### Ear-canal and middle-ear model.

As has been noted herein, a key aspect of the modeling approach includes the (1) addition of a third mass component M₃ to the middle-ear model from a previous version and (2) adjustment of the model parameter-search constraints (MPSC) to reduce the CHL estimation error. An important benefit of adding M₃ was to remove the influence of OME away from the third branch of the middle-ear model and toward the first branch, which caused R₁ to become more sensitive to effusion volume, thereby making R₁ a better predictor of CHL. The MPSC, which comprise mainly lower and upper bounds on middle-ear model parameters, influence the parameter-search toward more desirable model fits.

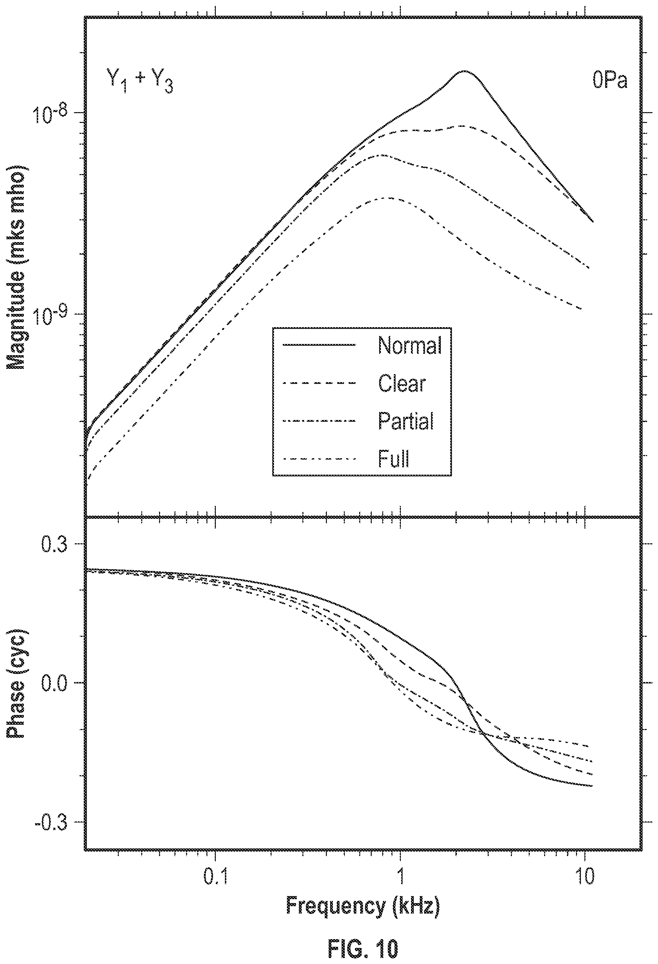

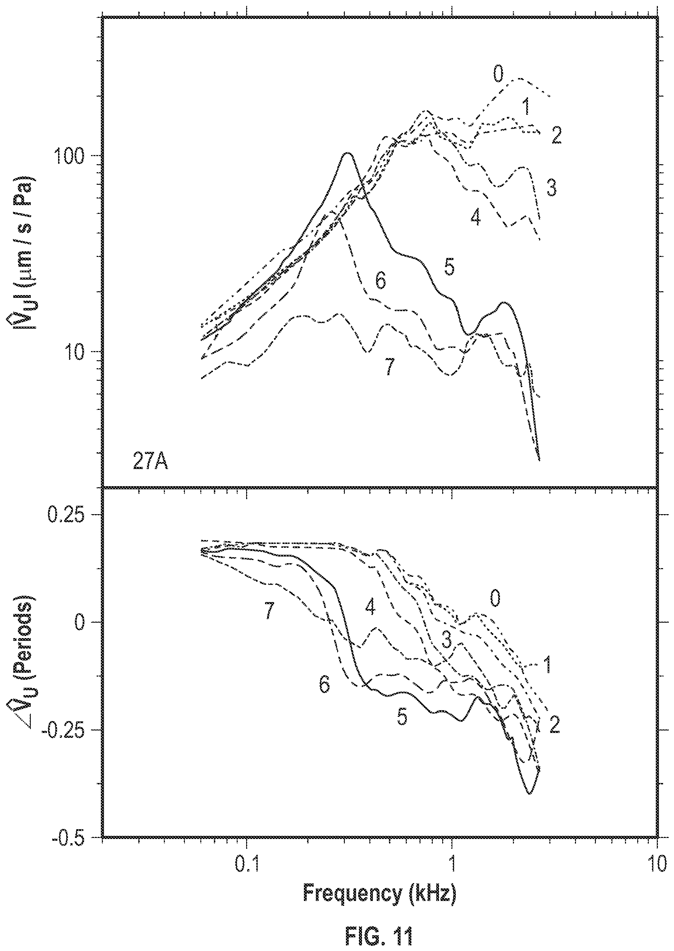

Although anatomical interpretation of middle-ear model components was not required for obtaining either CHL estimates or effusion-volume predictions, such interpretations are desirable for understanding underlying mechanisms. The strong correlation between the CHL estimates and the PTA supports the association of first branch with motion of the ossicular chain. On the other hand, the addition of M₃ causes *Y*₁ to no longer provide a good representation of umbo velocity (as it had in a previous model version) primarily because its resonance no longer shifts to lower frequencies with increasing effusion volume. In the present model, the trends observed in umbo velocity with increasing fluid volume are now better represented by *Y*ᵤₘ = *Y*₁ + *Y*₃. Comparison with measurements (see Figs. 10 and 11) indicate that *Y*ᵤₘ at 0Pa (Fig. 10) simulates umbo velocity (Fig. 11, curves numbered 0-4) better than either *Y*₁ alone (see Figs. 6A-6F) and better than *Y*ₘₑ = *Y*₁ + *Y*₂ + *Y*₃ (see Figs. 5A-5B). This observation suggests that the mode of TM motion represented by the third branch of the middle-ear model likely contributes to umbo velocity. In contrast, the second branch likely does not contribute to umbo velocity. So, the second branch apparently represents a mode of TM motion that does not contribute to motion of the ossicular chain.

The fact that the third branch was not included in an initial prediction of CHL makes its contribution to motion of the stapes unclear. Further insights into the association of model components with middle-ear anatomy may be acquired by considering the influence of static pressure on admittance.

The parameter-search methods currently used to fit the model to the measurements have been improved by selection of initial values from a set of values that are known to span the region of the parameter space of greatest interest. This method could be adapted to provide model fits to individual WAI measurements in a clinical setting in less than 10 seconds.

### Conductive hearing loss estimate.

Having a non-invasive and objective acoustic estimate of CHL in children with OME is clinically useful because obtaining pure tone thresholds and a PTA is difficult in young children and infants, where OME is most common.

The current approach disclosed herein to combining objective and non-invasive WAI measures with an electrical analog model of outer and middle ear mechanics results in CHL estimates within 3.2 dB of PTA. This is significant given that +/- 5 dB is considered the test-retest reliability of behavioral audiometry. In addition, this method could be easily implemented as an algorithm in software that could provide a CHL estimate in seconds after a WAI measurement was made, demonstrating clinical viability of this approach. It should be noted that the low prediction error in the present analysis may be partially due to the fact that adjustment of parameter-search constraints included consideration of CHL estimation.

### Diagnostic prediction performance.

The diagnostic prediction performance for the wet/dry decision (see, e.g., Table II, first row) is better than previously known. This improvement is due to the inclusion of regularization in the logistic-regression method, which reduces overfitting the training data.

The full/partial and clear/normal predictions have been improved by a combination of factors, including improvements to the middle-ear model and consideration of different static pressures. Current predictions utilizing the model and other aspects of the present disclosure are good enough to be clinically useful, which is an improvement over the need to have more invasive testing, which is difficult with certain age demographics. The machine-learning techniques disclosed herein will continue to improve the system and methods disclosed, as will testing on an extended bandwidth (e.g., from 8 kHz to 11 kHz).

Although the binomial classifier that has been developed for detection of ears in the UPR cluster has been based on a limited number of ears, it performed well on the entire set of ears with PTA by correctly identifying eight of the 11 ears in the UPR cluster. The three ears that were not detected by this classifier did not contribute much to the average prediction error because they all had PTA<5. Thus, the average CHL prediction error when using acoustic classification of UPR status was 3.7 dB, which is only 0.5 dB greater than the ideal error of 3.2 dB. The success of the UPR classifier on the training data suggests that predictions errors less than 4 dB may be achievable in a clinical setting. This is less than what test-retest reliability for the gold standard hearing assessment. behavioral pure tone audiometry.

The success of the CHL and effusion-volume estimation methods described herein are due in part by the nine-parameter model of middle-ear mechanics that decomposes WAI measurements into three modes of motion. The first mode appears to be strongly correlated to PTA, which supports the anatomical interpretation that this mode involves the ossicular chain and contributes to stapes motion. Comparisons with temporal-bone measurements suggest that the first and third modes both contribute to umbo motion while the second mode is likely to represent TM motion that is independent of umbo motion.

As noted herein, aspects of the disclosure include the development of systems and processes for the estimation of MECL and/or MEFV, such as in clinical applications. It is envisioned that the systems and/or processes, including the algorithms and machine learning networks, could be implemented with devices, such as WAI devices that are used in clinical settings. For example, the WAI tool could be used to non-invasively acquire the data from a patient in the clinic or other setting, and the tool or an associated network (e.g., a computer, server, processor, or the like) could quickly receive the acoustic data and provide the output information, which can include MECL and/or MEFV.

In addition, it should be appreciated that the aspects of the disclosure, including the development of the analog model, can be used for additional estimations of ear conditions and/or pathologies. For example, upon determination and fitting of parameters of ear mechanics via the analog model, said information could be correlated and compared with measured and assessed data from patients that include any number and/or type of ear condition, issue, and/or pathology. The machine learning network could be utilized to train the network to identify classifiers associated with any number of ear conditions, issues, and/or pathologies that could be identified using the WAI data to quickly and accurately provide results in a clinical setting that would negate the need for invasive testing on a patient.

In other words, the modeling and learning of the features of the present disclosure provide a near infinite basis for being able to assess, estimate, diagnose, provide treatment, or otherwise provide useful information related to ear conditions, issues, and/or pathologies for any number of patients, including those that may not be ideal for invasive and/or extensive testing, which is currently required. The results of the modeling and machine learning could be implemented into tools or otherwise associated with the tools, such as by receiving the data from the tools in a wired or wireless manner, to quickly provide the results and estimations for the conditions, features, issues, and/or pathologies of the patient's ear.

As will be understood, aspects and/or embodiments disclosed herein will utilize processors, memory, instructions, and the like, and will include a machine learning model or models to identify classifiers of aspects of ear conditions and/or pathologies. Machine learning (ML) is the study of computer algorithms that can improve automatically through experience and by the use of data. It is seen as a part of artificial intelligence. Machine learning algorithms build a model based on sample data, known as "training data", in order to make predictions or decisions without being explicitly programmed to do so.

While it is envisioned that generally any type of ML (e.g., supervised learning, unsupervised learning, semi-supervised learning, or reinforcement learning) can be utilized by any of the aspects and/or embodiments of the present disclosure utilize supervised learning. Supervised learning (SL) is the machine learning task of learning a function that maps an input to an output based on example input-output pairs. It infers a function from labeled training data consisting of a set of training examples. In supervised learning, each example is a pair consisting of an input object (typically a vector) and a desired output value (also called the supervisory signal). A supervised learning algorithm analyzes the training data and produces an inferred function, which can be used for mapping new examples. An optimal scenario will allow for the algorithm to correctly determine the class labels for unseen instances. This requires the learning algorithm to generalize from the training data to unseen situations in a "reasonable" way (see inductive bias). This statistical quality of an algorithm is measured through the so-called generalization error.

To solve a given problem of supervised learning, one has to perform the following steps: (1) Determine the type of training examples. Before doing anything else, the user should decide what kind of data is to be used as a training set. (2) Gather a training set. The training set needs to be representative of the real-world use of the function. Thus, a set of input objects is gathered, and corresponding outputs are also gathered, either from human experts or from measurements. (3) Determine the input feature representation of the learned function. The accuracy of the learned function depends strongly on how the input object is represented. Typically, the input object is transformed into a feature vector, which contains a number of features that are descriptive of the object. The number of features should not be too large, because of the curse of dimensionality; but should contain enough information to accurately predict the output. (4) Determine the structure of the learned function and corresponding learning algorithm. For example, the engineer may choose to use support-vector machines, regression analysis, or decision trees. (5) Complete the design. Run the learning algorithm on the gathered training set. Some supervised learning algorithms require the user to determine certain control parameters. These parameters may be adjusted by optimizing performance on a subset (called a validation set) of the training set, or via cross-validation. (6) Evaluate the accuracy of the learned function. After parameter adjustment and learning, the performance of the resulting function should be measured on a test set that is separate from the training set.

As will be understood, while generally any type of SL can be utilized, the example provided herein utilized three different classification algorithms to train the model, namely the support vector machine (SVM), k-Nearest Neighbors (k-NN), and classification ensemble (ENS).

Support-vector machines (SVMs, also support-vector networks) are supervised learning models with associated learning algorithms that analyze data for classification and regression analysis. Given a set of training examples, each marked as belonging to one of two categories, an SVM training algorithm builds a model that assigns new examples to one category or the other, making it a non-probabilistic binary linear classifier (although methods such as Platt scaling exist to use SVM in a probabilistic classification setting). SVM maps training examples to points in space so as to maximize the width of the gap between the two categories. New examples are then mapped into that same space and predicted to belong to a category based on which side of the gap they fall.

The k-nearest neighbors algorithm (k-NN) is a non-parametric classification method. k-NN is a type of classification where the function is only approximated locally, and all computation is deferred until function evaluation. Since this algorithm relies on distance for classification, if the features represent different physical units or come in vastly different scales then normalizing the training data can improve its accuracy dramatically.

Classification ensemble may also be referred to as ensemble learning. Ensemble methods use multiple learning algorithms to obtain better predictive performance than could be obtained from any of the constituent learning algorithms alone. Unlike a statistical ensemble in statistical mechanics, which is usually infinite, a machine learning ensemble consists of only a concrete finite set of alternative models, but typically allows for much more flexible structure to exist among those alternatives.

The trained model and the associated machine learning and application of the model will utilize processors, modules, memories, databases, networks, and potentially user interfaces to show the results and allow changes to be made.

In communications and computing, a computer readable medium is a medium capable of storing data in a format readable by a mechanical device. The term "non-transitory" is used herein to refer to computer readable media ("CRM") that store data for short periods or in the presence of power such as a memory device. It is envisioned that the WAI tools and/or associated processors for evaluating the data from the WAI tools could implement CRM.

One or more embodiments described herein can be implemented using programmatic modules, engines, or components. A programmatic module, engine, or component can include a program, a sub-routine, a portion of a program, or a software component or a hardware component capable of performing one or more stated tasks or functions. A module or component can exist on a hardware component independently of other modules or components. Alternatively, a module or component can be a shared element or process of other modules, programs, or machines.

The system will preferably include an intelligent control (i.e., a controller) and components for establishing communications. Examples of such a controller may be processing units alone or other subcomponents of computing devices. The controller can also include other components and can be implemented partially or entirely on a semiconductor (e.g., a field-programmable gate array ("FPGA")) chip, such as a chip developed through a register transfer level ("RTL") design process.

A processing unit, also called a processor, is an electronic circuit which performs operations on some external data source, usually memory or some other data stream. Nonlimiting examples of processors include a microprocessor, a microcontroller, an arithmetic logic unit ("ALU"), and most notably, a central processing unit ("CPU"). A CPU, also called a central processor or main processor, is the electronic circuitry within a computer that carries out the instructions of a computer program by performing the basic arithmetic, logic, controlling, and input/output ("I/O") operations specified by the instructions. Processing units are common in tablets, telephones, handheld devices, laptops, user displays, smart devices (TV, speaker, watch, etc.), and other computing devices.

As noted, the WAI tool itself could include a processor, and/or the tool could be connected, either wired or wirelessly to a separate system encompassing the processing unit.

The memory includes, in some embodiments, a program storage area and/or data storage area. The memory can comprise read-only memory ("ROM", an example of nonvolatile memory, meaning it does not lose data when it is not connected to a power source) or random access memory ("RAM", an example of volatile memory, meaning it will lose its data when not connected to a power source). Examples of volatile memory include static RAM ("SRAM"), dynamic RAM ("DRAM"), synchronous DRAM ("SDRAM"), etc. Examples of nonvolatile memory include electrically erasable programmable read only memory ("EEPROM"), flash memory, hard disks, SD cards, etc. In some embodiments, the processing unit, such as a processor, a microprocessor, or a microcontroller, is connected to the memory and executes software instructions that are capable of being stored in a RAM of the memory (e.g., during execution), a ROM of the memory (e.g., on a generally permanent basis), or another non-transitory computer readable medium such as another memory or a disc.

In the instant case, the memory could include the machine learned classifiers and analog models, so as to fit the parameters of the model and to quickly and accurately identify the results based on the trained classifiers.

Generally, the non-transitory computer readable medium operates under control of an operating system stored in the memory. The non-transitory computer readable medium implements a compiler which allows a software application written in a programming language such as COBOL, C++, FORTRAN, or any other known programming language to be translated into code readable by the central processing unit. After completion, the central processing unit accesses and manipulates data stored in the memory of the non-transitory computer readable medium using the relationships and logic dictated by the software application and generated using the compiler.

In one embodiment, the software application and the compiler are tangibly embodied in the computer-readable medium. When the instructions are read and executed by the non-transitory computer readable medium, the non-transitory computer readable medium performs the steps necessary to implement and/or use the present invention. A software application, operating instructions, and/or firmware (semi-permanent software programmed into read-only memory) may also be tangibly embodied in the memory and/or data communication devices, thereby making the software application a product or article of manufacture according to the present invention.

The database is a structured set of data typically held in a computer. The database, as well as data and information contained therein, need not reside in a single physical or electronic location. For example, the database may reside, at least in part, on a local storage device, in an external hard drive, on a database server connected to a network, on a cloud-based storage system, in a distributed ledger (such as those commonly used with blockchain technology), or the like.

It is envisioned that the machine learned model and any of the training of the same could include cloud computing. Cloud computing is a model of service delivery for enabling convenient, on-demand network access to a shared pool of configurable computing resources (e.g., networks, network bandwidth, servers, processing, memory, storage, applications, virtual machines, and services) that can be rapidly provisioned and released with minimal management effort or interaction with a provider of the service.

A cloud computing environment is service oriented with a focus on statelessness, low coupling, modularity, and semantic interoperability. At the heart of cloud computing is an infrastructure comprising a network of interconnected nodes.

As noted, the training model could be implemented on a user interface. The interface could also be a point on introduction of data, such as training data or test data to compare to the trained model for analysis. The results of the comparison could then be shown on a user interface.

A user interface is how the user interacts with a machine. The user interface can be a digital interface, a command-line interface, a graphical user interface ("GUI"), oral interface, virtual reality interface, or any other way a user can interact with a machine (user-machine interface). For example, the user interface ("UI") can include a combination of digital and analog input and/or output devices or any other type of UI input/output device required to achieve a desired level of control and monitoring for a device. Examples of input and/or output devices include computer mice, keyboards, touchscreens, knobs, dials, switches, buttons, speakers, microphones, LIDAR, RADAR, etc. Input(s) received from the UI can then be sent to a microcontroller to control operational aspects of a device.

The user interface module can include a display, which can act as an input and/or output device. More particularly, the display can be a liquid crystal display ("LCD"), a light-emitting diode ("LED") display, an organic LED ("OLED") display, an electroluminescent display ("ELD"), a surface-conduction electron emitter display ("SED"), a field-emission display ("FED"), a thin-film transistor ("TFT") LCD, a bistable cholesteric reflective display (i.e., e-paper), etc. The user interface also can be configured with a microcontroller to display conditions or data associated with the main device in real-time or substantially real-time.

Any components of the system could be connected via network or other communication protocol to transfer information, communicate with other systems, or provide other connectivity. In some embodiments, the network is, by way of example only, a wide area network ("WAN") such as a TCP/IP based network or a cellular network, a local area network ("LAN"), a neighborhood area network ("NAN"), a home area network ("HAN"), or a personal area network ("PAN") employing any of a variety of communication protocols, such as Wi-Fi, Bluetooth, ZigBee, near field communication ("NFC"), etc., although other types of networks are possible and are contemplated herein. The network typically allows communication between the communications module and the central location during moments of low-quality connections. Communications through the network can be protected using one or more encryption techniques, such as those techniques provided by the Advanced Encryption Standard (AES), which superseded the Data Encryption Standard (DES), the IEEE 802.1 standard for port-based network security, pre-shared key, Extensible Authentication Protocol ("EAP"), Wired Equivalent Privacy ("WEP"), Temporal Key Integrity Protocol ("TKIP"), Wi-Fi Protected Access ("WPA"), and the like.

Therefore, it has been shown that the model results provide a good description of the measured data. The presence of OME causes an increase in stiffness below 0.8 kHz and an increase in damping above 1 kHz, with larger changes resulting from larger volume effusions.

In addition, WAI, combined with the model provided herein, can estimate behavioral audiometric thresholds or CHL within a clinically meaningful margin of error. This is a significant finding given the challenges associated with behavioral audiometric testing in pediatric populations where OME is most common.

From the foregoing, it can be seen that the invention accomplishes at least all of the stated objectives.

## Claims

1. A computer-implemented method of estimating hearing loss, comprising:
obtaining an acoustic measurement from an ear canal;
modeling the acoustic measurement with an electric-analog model to obtain a model output;
transforming the model output to a measured absorbance to determine an averaged absorbance over a frequency range converted to decibels; and
training a machine learning network, wherein the training comprises:
acquiring measured hearing loss data;
fitting the parameters of the model such that the transformed model output correlates to the measured hearing data; and
identifying one or more classifiers of the transformed model output that provides an estimate of the hearing loss;
using the one or more classifiers to estimate hearing loss.

2. The method of claim 1, wherein the acoustic measurement comprises an impedance-based measurement.

3. The method of claim 2, wherein the impedance based measurement comprises a wideband acoustic immittance.

4. The method of claim 1, wherein the step of modeling the acoustic measurement further comprises a transmission line representing the ear canal terminated by the network.

5. The method of claim 4, wherein the network of the model comprises three parallel branches, with each branch comprising a stiffness, damping, and mass component, wherein the network represents mechanics of a tympanic membrane coupled to ossicles of an ear.

6. The method of claim 1, wherein the averaged absorbance over a frequency range is compared to a pure tone average to obtain the estimated hearing loss.

7. A computer-implemented method of estimating hearing loss, comprising:
obtaining an acoustic measurement from an ear canal;
modeling the acoustic measurement with an electric-analog model to obtain model outputs including absorbance levels and ossicular loss levels in decibels;
using a machine-learned network to identify ear type, wherein the machine-learned network comprises:
comparing ossicular loss level outputs to a pure tone average to identify classifiers; and
separating the classifiers into at least first and second ear types;
based upon the identified ear type, transforming the model outputs to estimate the hearing loss.

8. The method of claim 7, wherein the classifiers comprise:
a. a first identifier wherein the ossicular loss level outputs that are lower than the pure tone average; or
b. a second identifier wherein the ossicular loss level outputs that are larger than the pure tone average by a factor of at least two.

9. The method of claim 8, wherein the step of transforming the model outputs comprises combining the absorbance levels and ossicular loss levels to obtain an estimated hearing loss for the first identifiers.

10. The method of claim 9, wherein the step of transforming the model outputs comprises dividing the ossicular loss levels by two to obtain an estimated hearing loss for the second identifiers.

11. The method of claim 10, wherein the estimated hearing loss is determined by comparing the results to a known pure tone average.

12. The method of claim 7, wherein the acoustic measurement comprises an impedance-based measurement.

13. The method of claim 12, wherein the impedance based measurement comprises a wideband acoustic immittance.

14. The method of claim 7, wherein the step of modeling the acoustic measurement further comprises a transmission line representing the ear canal terminated by the network.

15. The method of claim 14, wherein the network of the model comprises three parallel branches, with each branch comprising a stiffness, damping, and mass component, wherein the network represents mechanics of a tympanic membrane coupled to ossicles of an ear.

16. A system for estimating hearing loss, comprising:
a device for obtaining an acoustic measurement from an ear canal;
the device including a computer readable medium configured to:
obtain the acoustic measurement from the ear canal;
model the acoustic measurement with an electric-analog model to obtain model outputs and training a machine-learned network, wherein the training comprises:
identifying values of the model output that correlate with measured or assessed data;
based upon machine-learned training, identify a classifier indicating an ear type, said machine-learned training including the steps of comparing ossicular loss level outputs to a pure tone average; and
based upon the identified ear type, transform the model outputs to estimate the hearing loss.

17. The system of claim 16, wherein the classifiers comprise:
a. a first identifier wherein the ossicular loss level outputs that are lower than the pure tone average; or
b. a second identifier wherein the ossicular loss level outputs that are larger than the pure tone average by a factor of at least two.

18. The system of claim 16, wherein the electric-analog model comprises a transmission line representing the ear canal terminated by the network.

19. The system of claim 18, wherein the network of the model comprises three parallel branches, with each branch comprising a stiffness, damping, and mass component, wherein the network represents mechanics of a tympanic membrane coupled to ossicles of an ear.

20. The system of claim 19, wherein the transmission line of the model represents an ear canal comprising a plurality of concatenated truncated-cone sections.

## Patentansprüche

1. Computerimplementiertes Verfahren zur Schätzung von Hörverlust, umfassend:
Erhalten einer akustischen Messung aus einem Gehörgang;
Modellieren der akustischen Messung mit einem elektrisch-analogen Modell zum Erhalten einer Modellausgabe;
Umwandeln der Modellausgabe in einen gemessenen Absorptionsgrad, um einen über einen in Dezibel umgewandelten Frequenzbereich gemittelten Absorptionsgrad zu bestimmen; und
Trainieren eines Netzwerks für maschinelles Lernen, wobei das Trainieren Folgendes umfasst:
Erfassen gemessener Hörverlustdaten;
Anpassen der Parameter des Modells, so dass die umgewandelte Modellausgabe mit den gemessenen Hördaten korreliert; und
Ermitteln von einem oder mehreren Klassifikatoren der umgewandelten Modellausgabe, die eine Schätzung des Hörverlusts bereitstellt;
Verwenden des einen oder der mehreren Klassifikatoren zur Schätzung von Hörverlust.

2. Verfahren nach Anspruch 1, wobei die akustische Messung eine impedanzbasierte Messung umfasst.

3. Verfahren nach Anspruch 2, wobei die impedanzbasierte Messung eine Breitband-Tympanometrie umfasst.

4. Verfahren nach Anspruch 1, wobei der Schritt des Modellierens der akustischen Messung ferner eine Übertragungsleitung umfasst, die den durch das Netzwerk abgeschlossenen Gehörgang darstellt.

5. Verfahren nach Anspruch 4, wobei das Netzwerk des Modells drei parallele Zweige umfasst, wobei jeder Zweig eine Steifigkeits-, Dämpfungs- und Massenkomponente umfasst, wobei das Netzwerk die Mechanik eines mit den Gehörknöchelchen eines Ohrs gekoppelten Trommelfells darstellt.

6. Verfahren nach Anspruch 1, wobei der über einen Frequenzbereich gemittelte Absorptionsgrad mit einem Reintonmittelwert verglichen wird, um den geschätzten Hörverlust zu erhalten.

7. Computerimplementiertes Verfahren zur Schätzung von Hörverlust, umfassend:
Erhalten einer akustischen Messung aus einem Gehörgang;
Modellieren der akustischen Messung mit einem elektrisch-analogen Modell zum Erhalten von Modellausgaben, die Absorptionsgradniveaus und die Schallleitungsschwerhörigkeitsniveaus in Dezibel beinhalten;
Verwenden eines durch maschinelles Lernen erhaltenen Netzwerks zum Ermitteln des Ohrtyps, wobei das durch maschinelles Lernen erhaltene Netzwerk Folgendes umfasst:
Vergleich der Schallleitungsschwerhörigkeitsniveau-Ausgaben mit einem Reintonmittelwert zur Ermittlung von Klassifikatoren; und
Aufteilen der Klassifikatoren in mindestens einen ersten und einen zweiten Ohrtyp;
Umwandeln, auf Basis des ermittelten Ohrtyps, der Modellausgaben zum Schätzen des Hörverlusts.

8. Verfahren nach Anspruch 7, wobei die Klassifikatoren Folgendes umfassen:
a. einen ersten Identifikator, bei dem die Schallleitungsschwerhörigkeitsniveau-Ausgaben niedriger als der Reintonmittelwert sind;
b. einen zweiten Identifikator, bei dem die Schallleitungsschwerhörigkeitsniveau-Ausgaben um den Faktor mindestens zwei größer als der Reintonmittelwert sind.

9. Verfahren nach Anspruch 8, wobei der Schritt des Umwandelns der Modellausgaben das Kombinieren der Absorptionsgradniveaus und der Schallleitungsschwerhörigkeitsniveaus zum Erhalten eines geschätzten Hörverlusts für die ersten Identifikatoren umfasst.

10. Verfahren nach Anspruch 9, wobei der Schritt des Umwandelns der Modellausgaben das Teilen der Schallleitungsschwerhörigkeitsniveaus durch zwei zum Erhalten eines geschätzten Hörverlusts für die zweiten Identifikatoren umfasst.

11. Verfahren nach Anspruch 10, wobei der geschätzte Hörverlust durch Vergleichen der Ergebnisse mit einem bekannten Reintonmittelwert bestimmt wird.

12. Verfahren nach Anspruch 7, wobei die akustische Messung eine impedanzbasierte Messung umfasst.

13. Verfahren nach Anspruch 12, wobei die impedanzbasierte Messung eine Breitband-Tympanometrie umfasst.

14. Verfahren nach Anspruch 7, wobei der Schritt des Modellierens der akustischen Messung ferner eine Übertragungsleitung umfasst, die den durch das Netzwerk abgeschlossenen Gehörgang darstellt.

15. Verfahren nach Anspruch 14, wobei das Netzwerk des Modells drei parallele Zweige umfasst, wobei jeder Zweig eine Steifigkeits-, Dämpfungs- und Massenkomponente umfasst, wobei das Netzwerk die Mechanik eines mit den Gehörknöchelchen eines Ohrs gekoppelten Trommelfells darstellt.

16. System zur Schätzung von Hörverlust, umfassend:
eine Vorrichtung zum Erhalten einer akustischen Messung aus einem Gehörgang;
wobei die Vorrichtung ein computerlesbares Medium beinhaltet, das konfiguriert ist zum:
Erhalten der akustischen Messung aus dem Gehörgang;
Modellieren der akustischen Messung mit einem elektrisch-analogen Modell zum Erhalten von Modellausgaben und Trainieren eines Netzwerks für maschinelles Lernen, wobei das Trainieren Folgendes umfasst:
Ermitteln von Werten der Modellausgabe, die mit gemessenen oder bewerteten Daten korrelieren;
auf Basis von Training durch maschinelles Lernen Ermitteln eines Klassifikators, der einen Ohrtyp anzeigt, wobei das genannte Training durch maschinelles Lernen die Schritte des Vergleichens von Schallleitungsschwerhörigkeitsniveau-Ausgaben mit einem Reintonmittelwert beinhaltet; und
Umwandeln, auf Basis des ermittelten Ohrtyps, der Modellausgaben zum Schätzen des Hörverlusts.

17. System nach Anspruch 16, wobei die Klassifikatoren Folgendes umfassen:
a. einen ersten Identifikator, wobei die Schallleitungsschwerhörigkeitsniveau-Ausgaben niedriger als der Reintonmittelwert sind;
b. einen zweiten Identifikator, bei dem die Schallleitungsschwerhörigkeitsniveau-Ausgaben um den Faktor mindestens zwei größer als der Reintonmittelwert sind.

18. System nach Anspruch 16, wobei das elektrisch-analoge Modell eine Übertragungsleitung umfasst, die den durch das Netzwerk abgeschlossenen Gehörgang darstellt.

19. System nach Anspruch 18, wobei das Netzwerk des Modells drei parallele Zweige umfasst, wobei jeder Zweig eine Steifigkeits-, Dämpfungs- und Massenkomponente umfasst, wobei das Netzwerk die Mechanik eines mit den Gehörknöchelchen eines Ohrs gekoppelten Trommelfells darstellt.

20. System nach Anspruch 19, wobei die Übertragungsleitung des Modells einen Gehörgang darstellt, der eine Mehrzahl von aneinandergereihten kegelstumpfförmigen Abschnitten umfasst.

## Revendications

1. Procédé mis en œuvre par ordinateur servant à l'estimation de la perte auditive, comprenant :
l'obtention d'une mesure acoustique provenant d'un conduit auditif ;
la modélisation de la mesure acoustique avec un modèle analogique électrique afin d'obtenir une sortie de modèle ;
la transformation de la sortie de modèle en une absorbance mesurée afin de déterminer une absorbance moyennée sur une plage de fréquences convertie en décibels ; et
l'entraînement d'un réseau à apprentissage machine, l'entraînement comprenant :
l'acquisition de données de perte auditive mesurées ;
l'adaptation des paramètres du modèle de telle sorte que la sortie de modèle transformée soit en corrélation avec les données auditives mesurées ; et
l'identification d'un ou de plusieurs classifieurs de la sortie de modèle transformée qui fournit une estimation de la perte auditive ;
l'utilisation des un ou plusieurs classifieurs pour estimer la perte auditive.

2. Procédé de la revendication 1, dans lequel la mesure acoustique comprend une mesure basée sur l'impédance.

3. Procédé de la revendication 2, dans lequel la mesure basée sur l'impédance comprend une immittance acoustique à large bande.

4. Procédé de la revendication 1, dans lequel l'étape de modélisation de la mesure acoustique comprend en outre une ligne de transmission représentant le conduit auditif terminé par le réseau.

5. Procédé de la revendication 4, dans lequel le réseau du modèle comprend trois branches parallèles, alors que chaque branche comprend un composant de rigidité, d'amortissement et de masse, dans lequel le réseau représente la mécanique d'une membrane tympanique couplée aux osselets d'une oreille.

6. Procédé de la revendication 1, dans lequel l'absorbance moyennée sur une plage de fréquences est comparée à une moyenne de sons purs afin d'obtenir la perte auditive estimée.

7. Procédé mis en œuvre par ordinateur servant à l'estimation de la perte auditive, comprenant :
l'obtention d'une mesure acoustique provenant d'un conduit auditif ;
la modélisation de la mesure acoustique avec un modèle analogique électrique afin d'obtenir des sorties de modèle, incluant des niveaux d'absorbance et des niveaux de pertes ossiculaires en décibels ;
l'utilisation d'un réseau obtenu par apprentissage machine pour identifier un type d'oreille, le réseau obtenu par apprentissage machine comprenant :
la comparaison des sorties de niveaux de pertes ossiculaires à une moyenne de sons purs afin d'identifier des classifieurs ; et
la séparation des classifieurs en au moins premier et deuxième types d'oreille ;
sur la base du type d'oreille ayant été identifié, la transformation des sorties de modèle pour estimer la perte auditive.

8. Procédé de la revendication 7, dans lequel les classifieurs comprennent :
a. un premier identifiant dans lequel les sorties de niveaux de pertes ossiculaires sont plus faibles que la moyenne de sons purs ; ou
b. un deuxième identifiant dans lequel les sorties de niveaux de pertes ossiculaires sont plus élevées que la moyenne de sons purs d'un facteur d'au moins deux.

9. Procédé de la revendication 8, dans lequel l'étape de transformation des sorties de modèle comprend le fait de combiner les niveaux d'absorbance et les niveaux de pertes ossiculaires afin d'obtenir une perte auditive estimée pour les premiers identifiants.

10. Procédé de la revendication 9, dans lequel l'étape de transformation des sorties de modèle comprend le fait de diviser les niveaux de pertes ossiculaires par deux afin d'obtenir une perte auditive estimée pour les deuxièmes identifiants.

11. Procédé de la revendication 10, dans lequel la perte auditive estimée est déterminée grâce à la comparaison des résultats à une moyenne de sons purs connue.

12. Procédé de la revendication 7, dans lequel la mesure acoustique comprend une mesure basée sur l'impédance.

13. Procédé de la revendication 12, dans lequel la mesure basée sur l'impédance comprend une immittance acoustique à large bande.

14. Procédé de la revendication 7, dans lequel l'étape de modélisation de la mesure acoustique comprend en outre une ligne de transmission représentant le conduit auditif terminé par le réseau.

15. Procédé de la revendication 14, dans lequel le réseau du modèle comprend trois branches parallèles, alors que chaque branche comprend un composant de rigidité, d'amortissement et de masse, dans lequel le réseau représente la mécanique d'une membrane tympanique couplée aux osselets d'une oreille.

16. Système pour l'estimation de la perte auditive, comprenant :
un dispositif pour obtenir une mesure acoustique provenant d'un conduit auditif ;
le dispositif incluant un support lisible par ordinateur configuré pour :
obtenir la mesure acoustique provenant du conduit auditif ;
modéliser la mesure acoustique avec un modèle analogique électrique afin d'obtenir des sorties de modèle et entraîner un réseau obtenu par apprentissage machine, dans lequel l'entraînement comprend :
l'identification de valeurs de la sortie de modèle qui sont en corrélation avec des données mesurées ou évaluées ;
sur la base de l'entraînement par apprentissage machine, identifier un classifieur indiquant un type d'oreille, ledit entraînement par apprentissage machine incluant les étapes consistant à comparer les sorties de niveau de pertes ossiculaires à une moyenne de sons purs ; et
sur la base du type d'oreille ayant été identifié, transformer les sorties de modèle pour estimer la perte auditive.

17. Système de la revendication 16, dans lequel les classifieurs comprennent :
a. un premier identifiant dans lequel les sorties de niveaux de pertes ossiculaires sont plus faibles que la moyenne de sons purs ; ou
b. un deuxième identifiant dans lequel les sorties de niveaux de pertes ossiculaires sont plus élevées que la moyenne de sons purs d'un facteur d'au moins deux.

18. Système de la revendication 16, dans lequel le modèle analogique électrique comprend une ligne de transmission représentant le conduit auditif terminé par le réseau

19. Système de la revendication 18, dans lequel le réseau du modèle comprend trois branches parallèles, alors que chaque branche comprend un composant de rigidité, d'amortissement et de masse, dans lequel le réseau représente la mécanique d'une membrane tympanique couplée aux osselets d'une oreille.

20. Système de la revendication 19, dans lequel la ligne de transmission du modèle représente un conduit auditif comprenant une pluralité de sections à cône tronqué en concaténation.
